# EUROPÄISCHE PATENTANMELDUNG

(11) **EP 2 705 878 A2**
(43) Veröffentlichungstag der Anmeldung: **12.03.2014**
(21) Anmeldenummer: 13179251.7
(22) Anmeldetag: 05.08.2013
(51) Int. Cl.: A61Q 5/00, A61K 8/92, A61K 8/37, A61K 8/892, A61K 8/891

(54) **Haarkur mit ausgewählten Esterölen, ausgewählten Silikonen und ausgewählten Acrylsäurederivaten mit geringem Wassergehalt**

(30) Priorität: 10.09.2012 DE 102012215965
(71) Anmelder: Henkel AG&Co. KGAA, 40589 Düsseldorf (DE)
(72) Erfinder: Battermann, Marlene, 21271 Asendorf (DE); Hippe, Thomas, 25482 Appen (DE)

(57) **Zusammenfassung**

Die Erfindung betrifft Haarbehandlungsmittel enthaltend ausgewählte Esteröle, ausgewählte Silikone und ausgewählte Acrylsäurederivate sowie die Verwendung dieser Mittel zur Haarbehandlung und zur Verbesserung der Lagerstabilität der Zusammensetzungen.

## Beschreibung

Die Erfindung betrifft Haarbehandlungsmittel enthaltend ausgewählte Esteröle, ausgewählte Silikone und ausgewählte Acrylsäurederivate sowie die Verwendung dieser Mittel zur Haarbehandlung.

Nicht zuletzt durch die starke Beanspruchung der Haare, beispielsweise durch das Färben oder Dauerwellen als auch durch die Reinigung der Haare mit Shampoos und durch Umweltbelastungen, nimmt die Bedeutung von Pflegeprodukten mit möglichst langanhaltender Wirkung zu.

Die bekannten Wirkstoffe können jedoch nicht alle Bedürfnisse in ausreichendem Maße abdecken. Es besteht daher weiterhin ein Bedarf nach Wirkstoffen bzw. Wirkstoffkombinationen für kosmetische Mittel mit guten pflegenden Eigenschaften. Insbesondere bei der Verwendung von kosmetischen Ölen wie Silikonen oder Glyceriden sowie natürlichen Ölen wie Sonnenblumenöl werden häufig instabile Zusammensetzungen erhalten. Diese Instabilitäten erweisen sich insbesondere als schwer handhabbar, wenn der Wassergehalt der Zusammensetzungen niedrig ist. In derartigen Formulierungen treten Verfärbungen auf und die Zusammensetzungen erweisen sich als nicht lagerstabil. Das Problem ist umso größer, wenn die Zusammensetzungen klar, farblos und transparent gewünscht werden. Aber auch bei gefärbten Zusammensetzungen verändert sich die Farbe. Beim Verbraucher wird dadurch der Eindruck erweckt, dass das Produkt nicht mehr zu verwenden ist. Außerdem wird auch der Duft der Zusammensetzungen nachteilig beeinflusst.

Die Aufgabe der vorliegenden Erfindung war es daher lagerstabile, insbesondere Farb- und Duftstabile Zusammensetzungen mit einem geringen Gehalt an Wasser zur Verfügung zu stellen. Überraschenderweise wurde nun jedoch gefunden, dass diese Aufgabe durch Zusammensetzungen enthaltend ausgewählte Esteröle, ausgewählte Silikone und ausgewählte Acrylsäurederivate mit einem Wassergehalt von höchstens 5,0 Gew.-% gelöst wird.

Gleichzeitig führt die Verwendung dieser Kombinationen zu überraschend guten Eigenschaften des behandelten Haares, insbesondere zu verbesserten Kämmbarkeiten und zu einer verbesserten Elastizität als auch zu einer deutlich gesteigerten Waschbeständigkeit gefärbten Haares, sowie zu einer längeren Haltbarkeit bei einer gleichzeitigen besseren Umformleistung bei Wellvorgängen wie Wasserwelle und Dauerwelle. Insbesondere wird jedoch der Glanz des damit behandelten Haares deutlich erhöht.

Diese positiven Eigenschaften werden sowohl bei einer typischen rinse-off als auch bei einer leave-on Applikation erhalten. In hervorragender Weise eignet sich die erfindungsgemäße Zusammensetzung auch für die Sprühapplikation als non-Aerosol. Hierbei wird gegenüber den bislang üblichen Produkten ein außerordentlich gleichmäßiges Sprühbild erzielt. Dadurch wird die gesamte Zusammensetzung sehr gleichmäßig auf dem Haar verteilt.

Ein erster Gegenstand der vorliegenden Erfindung ist daher eine Zusammensetzung zur Behandlung von keratinischen Fasern, enthaltend bezogen auf die gesamte Zusammensetzung des Mittels
a) mindestens ein Esteröl in einer Gesamtmenge von 0,01 bis 20,0 Gew.-%,
b) mindestens ein Silikon in einer Gesamtmenge von 0,01 bis 99 Gew.-%,
c) mindestens ein Acrylsäurederivat der Formel (c) 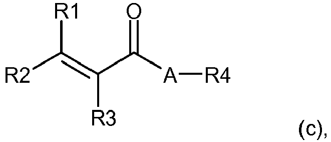 in welcher die Reste R1, R2, R3 jeweils unabhängig voneinander stehen für einen Rest ausgewählt aus Wasserstoff, einen linearen und/oder verzweigten, gesättigten und/oder ungesättigten C1 bis C30 Alkyl- oder Alkenylresten, insbesondere Methyl, Ethyl, Propyl, i-Propyl, Butyl, i-Butyl, t-Butyl, Pentyl, Amyl, i-Amyl, neo-Pentyl, Hexyl, i-Hexyl, 2-Ethylhexyl, Heptanyl, i-Heptanyl, Octanyl, i-Octanyl, Nonyl, Decyl, Lauryl, Myristyl, Aryl, Phenyl, Methoxyphenyl, Hydroxyphenyl, Methoxybenzyl, Hydroxybenzyl, Benzyl, Naphthyl oder-CN,
   A steht für O oder-NR5 und
   R4 sowie gegebenenfalls R5 stehen unabhängig voneinander für einen Rest ausgewählt aus Wasserstoff, einen linearen und/oder verzweigten, gesättigten und/oder ungesättigten C1 bis C30 Alkyl- oder Alkenylresten, insbesondere Methyl, Ethyl, Propyl, i-Propyl, Butyl, i-Butyl, t-Butyl, Pentyl, Amyl, i-Amyl, neo-Pentyl, Hexyl, i-Hexyl, 2-Ethylhexyl, Heptanyl, i-Heptanyl, Octanyl, i-Octanyl, Nonyl, Decyl, Lauryl, Myristyl, Aryl, Phenyl, Methoxyphenyl, Hydroxyphenyl, Methoxybenzyl, Hydroxybenzyl, Benzyl oder Naphthyl,
   in einer Gesamtmenge von 0,01 bis 5,0 Gew.-% und
d) Wasser in einer Gesamtmenge von 0 bis höchstens 5,0 Gew.-%.

Die Inhaltsstoffe a) bis d) werden nachfolgend detailliert beschrieben. Soweit nachstehend vom Wirkstoffkomplex (A) gesprochen wird, bezieht sich diese Aussage auf die in den erfindungsgemäßen Mitteln zwingend enthaltenen Inhaltsstoffe a) bis d).

Haarbehandlungsmittel im Sinne der vorliegenden Erfindung sind beispielsweise Haarkonditionierer, Haarsprays, Haarspülungen, Haarkuren, Haarpackungen, Haar-Tonics, Haarfestiger, Haarlegemittel, Haarstyling-Zubereitungen, Fönwell-Lotionen, Schaumfestiger, Haargele, Haarwachse oder deren Kombinationen. Bevorzugte erfindungsgemäße Mittel sind Shampoos, Konditioniermittel oder Haar-Tonics.

Unter Kämmbarkeit versteht sich erfindungsgemäß sowohl die Kämmbarkeit der nassen Faser, als auch die Kämmbarkeit der trockenen Faser. Als Maß für die Kämmbarkeit dient die aufgewendete Kämmarbeit oder die aufgewendete Kraft während des Kämmvorganges eines Faserkollektivs. Die Meßparameter können durch den Fachmann sensorisch beurteilt oder durch Messeinrichtungen quantifiziert werden.

Als Griff definiert sich die Taktilität eines Faserkollektivs, wobei der Fachmann sensorisch die Parameter Fülle und Geschmeidigkeit des Kollektivs fühlt und bewertet.

Unter Formgebung wird die Fähigkeit verstanden, einem Kollektiv zuvor behandelter keratinhaltiger Fasern, insbesondere menschlicher Haare, eine Formänderung zu verleihen. In der Haarkosmetik wird auch von Frisierbarkeit gesprochen.

Als kosmetische Träger eignen sich erfindungsgemäß besonders O/W - , W/O - und W/O/W - Emulsionen in Form von Cremes oder Gelen oder andere Zubereitungen, die insbesondere für die Anwendung auf dem Haar geeignet sind. Die kosmetischen Träger können insbesondere wässrig oder wässrig-alkoholisch sein.

Unter wässrig-alkoholischen kosmetischen Trägern sind im Sinne der vorliegenden Erfindung wässrige Lösungen enthaltend 3 bis 70 Gew.-% eines C₁-C₆-Alkohols, insbesondere Methanol, Ethanol bzw. Propanol, Isopropanol, Butanol, Isobutanol, tert.-Butanol, n-Pentanol, iso-Pentanole, n-Hexanol, iso-Hexanole, Glykol, Glycerin, 1,2-Pentandiol, 1,5-Pentandiol, 1,2-Hexandiol oder 1,6-Hexandiol zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Der Gesamtgehalt an Wasser in den erfindungsgemäßen Zusammensetzungen beträgt höchstens 5,0 Gew.-% Bevorzugt ist ein Gehalt von höchstens 3,0 Gew.-% Wasser. Besonders bevorzugt ist ein Gehalt von höchstens 1,0 Gew.-% Wasser. Höchst bevorzugt ist ein Wassergehalt von höchstens 0,3 Gew.-% und am bevorzugtesten ist ein Wassergehalt von höchstens 0,1 Gew.-%. Zur Bestimmung des Wassergehaltes in den erfindungsgemäßen Zusammensetzungen ist auch das Wasser zu berücksichtigen, welches gegebenenfalls in den eingesetzten Rohstoffen enthalten ist. Daraus folgt, dass alle Rohstoffe, welche einen hohen Gehalt an Wasser enthalten, nicht unbedingt in den erfindungsgemäßen Zusammensetzungen verwendet werden sollten. Erfindungsgemäß möglich ist es jedoch, einem solchen Rohstoff, wenn er nicht mit einem reduzierten Wassergehalt oder nahezu wasserfrei erhältlich ist, das Wasser vor der Verwendung in der erfindungsgemäßen Zusammensetzung durch bekannte Verfahren zu entziehen. Hierzu eignet sich beispielsweise eine Gefriertrocknung, eine Destillation etc.

Als Inhaltstoff a) enthalten die erfindungsgemäßen Mittel mindestens ein kosmetisches Öl ausgewählt aus den Esterölen. Bevorzugt weisen die Esteröle einen Schmelzpunkt kleiner als 50 °C, besonders bevorzugt kleiner als 45 °C, ganz besonders bevorzugt kleiner als 40 °C, höchst bevorzugt kleiner als 35 °C und am bevorzugtesten sind die kosmetischen Esteröle bei einer Temperatur kleiner als 30 °C fließfähig. Die Esteröle sind wie folgt definiert:
Unter Esterölen sind zu verstehen die Ester von C₆ - C₃₀ - Fettsäuren mit C₂ - C₃₀ - Fettalkoholen

Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).

Selbstverständlich können die Esteröle auch mit Etyhlenoxid, Propylenoxid oder Mischungen aus Ethylenoxid und Propylenoxid alkoxiliert sein. Die Alkoxylierung kann dabei sowohl am Fettalkoholpart als auch am Fettsäurepart als auch an beiden Teilen der Esteröle zu finden sein.

Bevorzugt ist erfindungsgemäß jedoch, wenn zunächst der Fettalkohol alkoxyliert wurde und anschließend mit Fettsäure verestert wurde. In der Formel (D4-II) sind allgemein diese Verbindungen dargestellt. 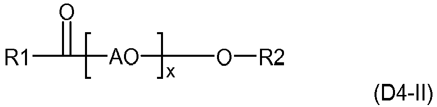 R1 steht hierbei für einen gesättigten oder ungesättigten, verzweigten oder unverzweigten, cyclischen gesättigten cyclischen ungesättigten Acylrest mit 6 bis 30 Kohlenstoffatomen,
AO steht für Ethylenoxid, Propylenoxid oder Butylenoxid,
X steht für eine Zahl zwischen 1 und 200, vorzugsweise 1 und 100, besonders bevorzugt zwischen 1 und 50, ganz besonders bevorzugt zwischen 1 und 20, höchst bevorzugt zwischen 1 und 10 und am bevorzugtesten zwischen 1 und 5,
R2 steht für einen gesättigten oder ungesättigten, verzweigten oder unverzweigten, cyclischen gesättigten cyclischen ungesättigten Alkyl-, Alkenyl-, Alkinyl-, Phenyl- oder Benzylrest mit 6 bis 30 Kohlenstoffatomen. Beispiele für eingesetzte Fettsäurenanteile als Rest R1 in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile als Rest R2 in den Esterölen sind Benzylalkohol, Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Ein erfindungsgemäß besonders bevorzugtes Esteröl ist beispielsweise unter der INCI - Bezeichnung PPG-3 Benzyl Ether Myristate erhältlich.

Weiterhin sind unter Esterölen zu verstehen:
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat, sowie
- symmetrische, unsymmetrische oder cyclische Ester der Kohlensäure mit Fettalkoholen, beispielsweise Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.
- pflanzliche Öle wie beispielsweise Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Haselnussöl, Holundersamenöl, Johannesbeersamenöl, Jojobaöl, Kakaobutter, Leinöl, Macadamianussöl, Maiskeimöl, Mandelöl, Marulaöl, Nachtkerzenöl, Olivenöl, Orangenöl, Palmöl, Pfirsichkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl Weizenkeimöl, Wildrosenöl und die flüssigen Anteile des Kokosöls.
- natürliche Öle sind beispielsweise andere Triglyceridöle, die nicht auf einer pflanzlichen Basis gewonnen werden wie beipsielsweise die flüssigen Anteile des Rindertalgs sowie synthetisch hergestellte Triglyceridöle.

Mit besonderem Vorzug werden die Esteröle ausgewählt aus den pflanzlichen und/oder natürlichen Ölen. Höchst bevorzugt sind die Esteröle ausgewählt aus Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Haselnussöl, Holundersamenöl, Johannesbeersamenöl, Jojobaöl, Kakaobutter, Leinöl, Macadamianussöl, Maiskeimöl, Mandelöl, Marulaöl, Nachtkerzenöl, Olivenöl, Orangenöl, Palmöl, Pfirsichkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl Weizenkeimöl, Wildrosenöl und den flüssigen Anteilen des Kokosöls sowie Mischungen der zuvor genannten Esteröle. Am bevorzugtesten sind die Esteröle ausgewählt aus Amaranthsamenöl, Aprikosenkernöl, Arganöl, Avocadoöl, Babassuöl, Baumwollsaatöl, Granatapfelkernöl, Grapefruitsamenöl, Haselnussöl, Holundersamenöl, Johannesbeersamenöl, Jojobaöl, Kakaobutter, Macadamianussöl, Maiskeimöl, Mandelöl, Marulaöl, Nachtkerzenöl, Olivenöl, Orangenöl, Sanddornfruchtfleischöl, Sanddornkernöl, Sesamöl, Sheabutter, Sojaöl, Sonnenblumenöl, Traubenkernöl, Walnußöl Weizenkeimöl, Wildrosenöl und deren Mischungen. Die Esteröle werden in den erfindungsgemäßen Mitteln in einer Menge von 0,01 bis 20 Gew.-%, bevorzugt 0,01 bis 10,0 Gew.-%, besonders bevorzugt 0,01 bis 7,5 Gew.-%, höchst bevorzugt von 0,1 bis 5,0 Gew.-% verwendet. Selbstverständlich ist es erfindungsgemäß auch möglich mehrere Esteröle gleichzeitig zu verwenden.

Weitere erfindungsgemäße Öle, welche gegebenenfalls mit den Esterölen und den Silikonen gemeinsam verwendet werden können, sind:
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.

Diese letztgenannten Öle werden in den erfindungsgemäßen Mitteln in einer Menge von 0,01 bis 20 Gew.-%, bevorzugt 0,01 bis 10,0 Gew.-%, besonders bevorzugt 0,01 bis 7,5 Gew.-%, höchst bevorzugt von 0,1 bis 5,0 Gew.-% verwendet. Selbstverständlich ist es erfindungsgemäß auch möglich mehrere dieser letztgenannten Öle gleichzeitig zu verwenden.

Weiterhin ist der Inhaltsstoff b) der erfindungsgemäßen Zusammensetzungen mindestens ein Silikonöl. Die Auswahl des Silikonöles erfolgt hierbei aus den dem Fachmann bekannten Silikonölen. Das Silikonöl wird üblicherweise ausgewählt aus der Gruppe der Dimethicone, zu welchen auch die Cyclomethicone zu rechnen sind, und/oder der Gruppe der Dimethiconole und/oder der Gruppe der aminofunktionellen Silikone. Wenngleich zu den Silikonölen auch die Dimethiconcopolyole zu rechnen sind, kann es erfindungsgemäß bevorzugt sein, kein Dimethiconcopolyol auszuwählen.

Erfindungsgemäß bevorzugt ist es, wenn mindestens zwei unterschiedliche Silikone verwendet werden. Am bevorzugtesten werden Dimethicone, Dimethiconole, Cyclomethicone, und Silikone der Formel (Si-5) sowie deren Mischungen verwendet. Die Silikone werden in einer Gesamtmenge von mindestens 0,01 bis 99,0 Gew.-% verwendet. Vorzugsweise werden insgesamt 0,01 bis 90,0 Gew.-% an Silikonen verwendet. Besonders bevorzugt werden die Silikone in einer Gesamtmenge von 0,1 bis 85 Gew.-% jeweils bezogen auf die gesamt Zusammensetzung verwendet. Innerhalb dieser Gesamtmenge an Silikon werden die einzelnen zu verwendenden Silikone innerhalb der bei der Beschreibung der einzelnen Silikone genannten Mengen verwendet.

Die erfindungsgemäßen Dimethicone können sowohl linear als auch verzweigt als auch cyclisch oder cyclisch und verzweigt sein. Lineare Dimethicone können durch die folgende Strukturformel (Si1) dargestellt werden:

(SiR'₃) - O - (SiR²₂ - O - )ₓ - (SiR'₃) (Si1)

Verzweigte Dimethicone können durch die Strukturformel (Si1.1) dargestellt werden:

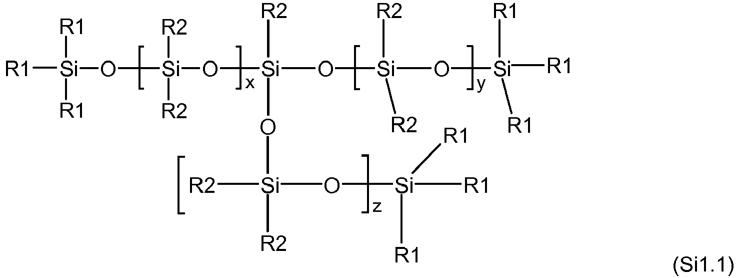

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs. Höchst bevorzugt sind Viskositäten um den Bereich von etwa 60.000 cPs herum. Beispielhaft sei hier auf das Produkt "Dow Corning 200 mit 60000cSt" verwiesen.

Besonders bevorzugte erfindungsgemäße kosmetische oder dermatologische Zubereitungen sind dadurch gekennzeichnet, dass sie mindestens ein Silikon der Formel (Si1.2)

(CH₃)₃Si-[O-Si(CH₃)₂]ₓ-O-Si(CH₃)₃ (Si1.2),

enthalten, in der x für eine Zahl von 0 bis 100, vorzugsweise von 0 bis 50, weiter bevorzugt von 0 bis 20 und insbesondere 0 bis 10, steht.

Die Dimethicone (Si1) sind in den erfindungsgemäßen Zusammensetzungen in Mengen von 0,01 bis 10 Gew.-%, vorzugsweise 0,01 bis 8 Gew.-%, besonders bevorzugt 0,1 bis 7,5 Gew.-% und insbesondere 0,1 bis 5 Gew.-% bezogen auf die gesamte Zusammensetzung enthalten. Schließlich werden unter den Silikonverbindungen die Dimethiconole (Si8) verstanden. Die erfindungsgemäßen Dimethiconole können sowohl linear als auch verzweigt als auch cyclisch oder cyclisch und verzweigt sein. Lineare Dimethiconole können durch die folgende Strukturformel (Si8 - I) dargestellt werden:

(SiOHR¹₂) - O - (SiR²₂ - O -)ₓ - (SiOHR¹₂) (Si8 - I)

Verzweigte Dimethiconole können durch die Strukturformel (Si8 - II) dargestellt werden:

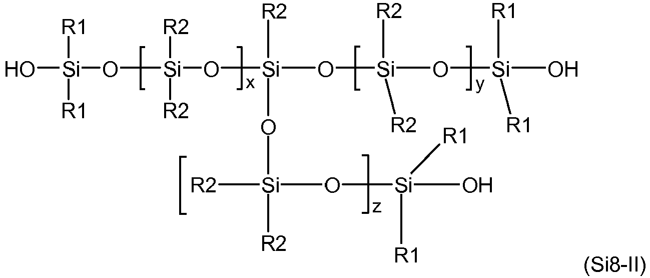

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Die Zahlen x, y und z sind ganze Zahlen und laufen jeweils unabhängig voneinander von 0 bis 50.000. Die Molgewichte der Dimethiconole liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Als Beispiele für derartige Produkte werden die folgenden Handelsprodukte genannt: Dow Corning 1-1254 Fluid, Dow Corning 2-9023 Fluid, Dow Corning 2-9026 Fluid, Abil OSW 5 (Degussa Care Specialties), Dow Corning 1401 Fluid, Dow Corning 1403 Fluid, Dow Corning 1501 Fluid, Dow Corning 1784 HVF Emulsion, Dow Corning 9546 Silicone Elastomer Blend, SM555, SM2725, SM2765, SM2785 (alle vier zuvor genannten GE Silicones), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH). Die Dimethiconole (Si8) sind in den erfindungsgemäßen Zusammensetzungen in Mengen von 0,01 bis 95 Gew.-%, vorzugsweise 0,01 bis 70,0 Gew.-%, besonders bevorzugt 0,1 bis 50,0 Gew.-% und insbesondere 0,1 bis 35,0 Gew.-% an Dimethiconol bezogen auf die Zusammensetzung. Auch die nach INCI als Cyclomethicone bezeichneten cyclischen Dimethicone sind erfindungsgemäß mit Vorzug einsetzbar. Hier sind erfindungsgemäße kosmetische oder dermatologische Zubereitungen bevorzugt, die mindestens ein Silikon der Formel (Si-4) 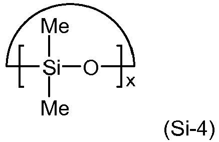 enthalten, in der x für eine Zahl von 3 bis 200, vorzugsweise von 3 bis 10, weiter bevorzugt von 3 bis 7 und insbesondere 3, 4, 5 oder 6, steht.

Die Cyclomethicone (Si-4) sind in den erfindungsgemäßen Zusammensetzungen in Mengen von 0,1 bis 95 Gew.-%, vorzugsweise 0,1 bis 85,0 Gew.-%, besonders bevorzugt 0,1 bis 75,0 Gew.-% und insbesondere 1,0 bis 70,0 Gew.-% an Cyclomethicone bezogen auf die Zusammensetzung. Erfindungsgemäß ebenfalls bevorzugte Mittel sind dadurch gekennzeichnet, dass sie mindestens ein Silikon der Formel (Si-5)

R₃Si-[O-SiR₂]ₓ-(CH₂)ₙ-[O-SiR₂]_{y}-O-SiR₃ (Si-5),

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht. Höchst bevorzugt ist insbesondere die Verbindung mit der Bezeichnung Octamethyltrisiloxane.

Die Silikone der Formel (Si-5) sind in den erfindungsgemäßen Zusammensetzungen in Mengen von 0,1 bis 75 Gew.-%, vorzugsweise 0,1 bis 60,0 Gew.-%, besonders bevorzugt 0,1 bis 50,0 Gew.-% und insbesondere 1,0 bis 40,0 Gew.-% an bezogen auf die Zusammensetzung, enthalten. In einer besonders bevorzugten Ausführungsform des erfindungsgemäßen Wirkstoffkomplexes enthalten die erfindungsgemäßen Mittel bevorzugt mindestens ein aminofunktionelles Silikon. Solche Silikone können z.B. durch die Formel (Si-2)

M(RₐQ_{b}SiO(_{4-a-b})_{/2})ₓR_{c}SiO_{(4-c)/2})_{y}M (Si-2)

Beschrieben werden, wobei in der obigen Formel
R ein Kohlenwasserstoff oder ein Kohlenwasserstoffrest mit 1 bis etwa 6 Kohlenstoffatomen ist,
Q ein polarer Rest der allgemeinen Formel -R'HZ ist,
   worin
   R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und
   Z ein organischer, aminofunktioneller Rest ist, der mindestens eine aminofunktionelle Gruppe enthält;
a Werte im Bereich von etwa 0 bis etwa 2 annimmt,
b Werte im Bereich von etwa 1 bis etwa 3 annimmt,
a + b kleiner als oder gleich 3 ist, und
c eine Zahl im Bereich von etwa 1 bis etwa 3 ist, und
x eine Zahl im Bereich von 1 bis etwa 2.000, vorzugsweise von etwa 3 bis etwa 50 und am bevorzugtesten von etwa 3 bis etwa 25 ist, und
y eine Zahl im Bereich von etwa 20 bis etwa 10.000, vorzugsweise von etwa 125 bis etwa 10.000 und am bevorzugtesten von etwa 150 bis etwa 1.000 ist, und
M eine geeignete Silikon-Endgruppe ist, wie sie im Stande der Technik bekannt ist, vorzugsweise Trimethylsiloxy.

Z ist gemäß Formel (Si-2) ein organischer, aminofunktioneller Rest, enthaltend mindestens eine funktionelle Aminogruppe. Eine mögliche Formel für besagtes Z ist NH(CH₂)_{z}NH₂, worin z eine ganze Zahl von größer gleich 1 ist. Eine andere mögliche Formel für besagtes Z ist -NH(CH₂)_{z}(CH ₂)_{zz}NH, worin sowohl z als auch zz unabhängig voneinander eine ganze Zahl von größer gleich 1 sind, wobei diese Struktur Diamino-Ringstrukturen umfasst, wie Piperazinyl. Besagtes Z ist am bevorzugtesten ein -NHCH₂CH ₂NH₂-Rest. Eine andere mögliche Formel für besagtes Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist. Q gemäß Formel (Si-2) ist am bevorzugtesten ein polarer aminofunktioneller Rest der Formel - CH₂CH₂CH₂NHCH₂CH₂NH₂.

In der Formel (Si-2) nimmt a Werte im Bereich von 0 bis 2 an, b nimmt Werte im Bereich von 2 bis 3 an, a + b ist kleiner als oder gleich 3, und c ist eine Zahl im Bereich von 1 bis 3. Erfindungsgemäß geeignet sind kationische Silikonöle wie beispielsweise die im Handel erhältlichen Produkte Dow Corning (DC) 929 Emulsion, DC 2-2078, DC 5-7113, SM-2059 (General Electric) sowie SLM-55067 (Wacker).

Besonders bevorzugte erfindungsgemäße Mittel sind dadurch gekennzeichnet, dass sie mindestens es ein aminofunktionelles Silikon der Formel (Si3-a) 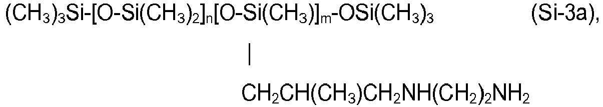 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet und sind beispielsweise unter der Bezeichnung Q2-7224 (Hersteller: Dow Corning; ein stabilisiertes Trimethylsilylamodimethicon) erhältlich.

Besonders bevorzugt sind auch erfindungsgemäße Mittel, die mindestens ein aminofunktionelles Silikon der Formel (Si-3b) 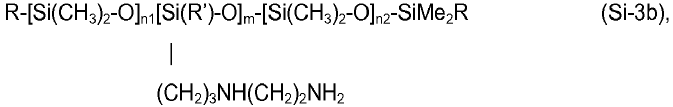 enthalten, worin
R für -OH, eine (gegebenenfalls ethoxylierte und/oder propoxylierte) (C₁ bis C₂₀)-Alkoxygruppe oder eine -CH₃-Gruppe steht,
R' für -OH, eine (C₁ bis C₂₀)-Alkoxygruppe oder eine -CH₃-Gruppe und
m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), bezeichnet.

Geeignete diquaternäre Silikone sind ausgewählt aus Verbindungen der allgemeinen Formel (Si3c)

[R¹R²R³N⁺ - A - SiR⁷R⁸ - (O-SiR⁹R¹⁰)ₙ- O - SiR¹¹R¹² - A - N⁺R⁴R-⁵R⁶] 2X (Si3c)

wobei die Reste R1 bis R6 unabhängig voneinander C1-bis C22-Alkylreste bedeuten, welche Hydroxygruppen enthalten können und wobei vorzugsweise mindestens einer der Reste mindestens 8 C-Atome aufweist und die übrigen Reste 1 bis 4 C-Atome aufweisen,
die Reste R7 bis R12 unabhängig voneinander gleich oder verschieden sind und C1-bis C10-Alkyl oder Phenyl bedeuten, A eine divalente organische Verbindungsgruppe bedeutet,
n eine Zahl von 0 bis 200, vorzugsweise von 10 bis 120, besonders bevorzugt von 10 bis 40 ist, und X⁻ ein Anion ist.

Die divalente Verbindungsgruppe ist vorzugsweise eine C1-bis C12-Alkylen-oder Alkoxyalkylengruppe, die mit einer oder mehreren Hydroxylgruppen substituiert sein kann. Besonders bevorzugt ist die Gruppe -(CH_{z})₃-O-CH₂-CH(OH)-CH₂-.

Das Anion X⁻ kann ein Halogenidion, ein Acetat, ein organisches Carboxylat oder eine Verbindung der allgemeinen Formel RSO₃⁻ sein, worin R die Bedeutung von C1-bis C4-Alkylresten hat.

Ein ganz besonders bevorzugtes aminofunktionales Aminosilikon ist mindestens ein 4-morpholinomethyl-substituiertes Silikon der Formel (V) 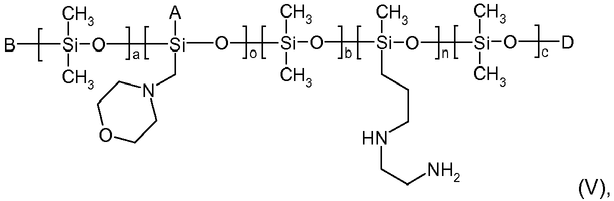 in der
A für eine über ein -O- gebundene Struktureinheit (I), (II) oder (III) 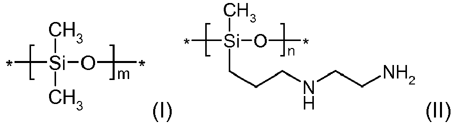 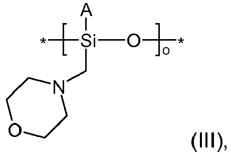 oder einen über ein -O- gebundenen oligomeren oder polymeren Rest enthaltend Struktureinheiten der Formeln (I), (II) oder (III) oder die Hälfte eines verbindenden O-Atoms zu einer Struktureinheit (III) oder für-OH steht,
* für eine Bindung zu einer der Struktureinheiten (I), (II) oder (III) oder für eine Endgruppe B (Si-gebunden) oder D (O-gebunden) steht,
B für eine Gruppe -OH, -O-Si(CH₃)₃,-O-Si(CH₃)₂OH ,-O-Si(CH₃)₂OCH₃ steht,
D für eine Gruppe -H, -Si(CH₃)₃,-Si(CH₃)₂OH, -Si(CH₃)₂OCH₃ steht,
a, b und c für ganze Zahlen zwischen 0 und 1000 stehen, mit der Maßgabe a + b + c > 0
m, n und o für ganze Zahlen zwischen 1 und 1000 stehen.

Ein bevorzugtes diquaternäres Silikon hat die allgemeine Formel (Si3d)

[RN+Me₂ - A - (SiMe₂O)ₙ - SiMe₂ - A - N⁺Me₂R] 2 CH₃COO⁻ (Si3d),

wobei A die Gruppe -(CH₂)₃- O - CH₂ - CH(OH) - CH₂ -ist,

R ein Alkylrest mit mindestens 8 C-Atomen und n eine Zahl von 10 bis 120 ist.

Geeignete Silikonpolymere mit zwei endständigen, quaternären Ammoniumgruppen sind unter der INCI-Bezeichnung Quaternium-80 bekannt. Hierbei handelt es sich um Dimethylsiloxane mit zwei endständigen Trialkylammoniumgruppen. Derartige diquaternäre Polydimethylsiloxane werden von der Firma Evonik unter den Handelsnamen Abil^{®} Quat 3270, 3272 und 3474 vertrieben. Erfindungsgemäß bevorzugte Haarbehandlungsmittel sind dadurch gekennzeichnet, dass sie, bezogen auf ihr Gewicht, 0,01 bis 10 Gew.-%, vorzugsweise 0,01 bis 8 Gew.-%, besonders bevorzugt 0,1 bis 7,5 Gew.-% und insbesondere 0,2 bis 5 Gew.-% aminofunktionelle(s) Silikon(e) und/oder diquaternäres Silikon enthalten.

Ein weiteres erfindungsgemäßes Silikon mit Aminofunktionen sind Polyammonium-Polysiloxan Verbindungen. Die Polyammonium-Polysiloxan Verbindungen können beispielsweise unter der Handelsbezeichnung Baysilone® von GE Bayer Silicones bezogen werden. Die Produkte mit den Bezeichnungen Baysilone TP 3911, SME 253 und SFE 839 sind dabei bevorzugt. Ganz besonders bevorzugt ist die Verwendung von Baysilone TP 3911 als Wirkkomponente der erfindungsgemäßen Zusammensetzungen. Die Polyammonium-Polysiloxan Verbindungen werden in dem erfindungsgemäßen Zusammensetzungen in einer Menge von 0,01 bis 10 Gew.-% , vorzugsweise 0,01 bis 7,5, besonders bevorzugt 0,01 bis 5,0 Gew.-%, ganz besonders bevorzugt von 0,05 bis 2,5 Gew.-% jeweils in Bezug auf die Gesamtzusammensetzung verwendet.

Die EP 1887024 A1 beschreibt neuartige kationische aminofunktionelle Silikone, welche insbesondere den Glanz in Mitteln zur Pflege von Oberflächen, beispielsweise menschlichen Haaren, verbessern. Diese kationischen Silikonpolymere zeichnen sich dadurch aus, dass sie ein Silikongerüst sowie mindestens einen Polyetherteil und weiterhin mindestens einen Teil mit Ammoniumstruktur aufweisen. Beispiele für die bevorzugten kationischen Silikonpolymere im Sinne der vorliegenden Erfindung sind neben den Verbindungen der zuvor genannten EP 1887024 A1 weiterhin insbesondere die Verbindungen mit den INCI - Bezeichnungen: Silicone Quaternium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-17, Silicone Quaternium-18, Silicone Quaternium-20, Silicone Quaternium-21, Silicone Quaternium-22 sowie Silicone Quaternium-2 Panthenol Succinate und Silicone Quaternium-16/Glycidyl Dimethicone Crosspolymer. Am bevorzugtesten ist insbesondere Silicone Quaternium-22. Dieser Rohstoff wird beispielsweise von der Firma Evonik unter der Handelsbezeichnung Abil^{®} T-Quat 60 vertrieben.

Ein weiteres erfindungsgemäß besonders bevorzugtes Aminosilikon entspricht der folgenden Formel:

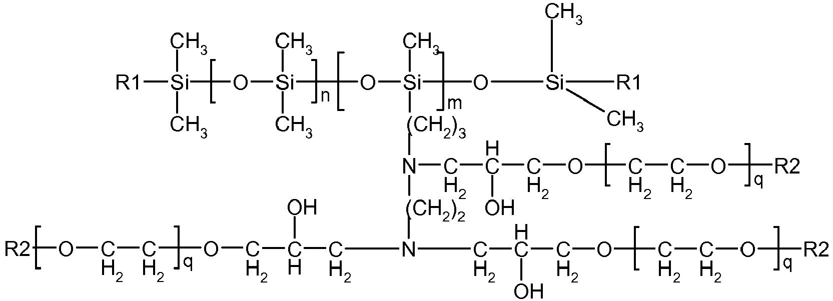

in welcher R1 steht für eine Methyl-, Ethyl-, Hydroxy-, Methoxy- oder Ethoxygruppe,

R2, steht für einen geradkettigen oder verzweigten C8 bis C24 Alkyl- oder Alkylenrest, vorzugsweise einen geradkettigen oder verzweigten C9 bis C22 Alkyl- oder Alkenylrest, besonders bevorzugt einen geradkettigen oder verzweigten C11 bis C18 Alkyl- oder Alkenylrest, höchst bevorzugt einen entsprechenden Alkylrest,

n und m jeweils für ganze Zahlen von 1 bis 1000 stehen und q steht jeweils für eine ganze Zahl von 2 bis 50, bevorzugt 4 bis 30, besonders bevorzugt 4 bis 18 und höchst bevorzugt von 4 bis 12.

Das Molekulargewicht derartiger Verbindungen beträgt 15.000 bis 2.000.000, gemessen mit einem Brookfield Rotationsviskosimeter RV, Spindel 5, bei 20 °C. Bevorzugt beträgt das Molgewicht 30.000 bis 1.750.000 und besonders bevorzugt 50.000 bis 1.500.000. Der Stickstoffgehalt der erfindungsgemäßen Silikone beträgt 0,03 bis 4,2 Gew.-%, bevorzugt 0,1 bis 2,8 Gew.-% und höchst bevorzugt 0,16 bis 1,4 Gew.-%. Erfindungsgemäße aminofunktionelle kationische Silikone der obigen Formel können beispielsweise von der Fa. Clariant bezogen werden. Ein erfindungsgemäß höchst bevorzugtes Produkt ist unter der INCI-Bezeichnung "Trideceth-9-Amodimethicone and Trideceth-12" im Handel erhältlich.

Die kationischen aminofunktionellen Silikonpolymere der zuvor beschriebenen Formel sind in den erfindungsgemäßen Zusammensetzungen in Mengen von 0,01 bis 5 Gew.%, bevorzugt in Mengen von 0,05 bis 5 Gew.-% und ganz besonders bevorzugt in Mengen von 0,1 bis 5 Gew.-% enthalten. Die allerbesten Ergebnisse werden dabei mit Mengen von 0,1 bis 2,5 Gew.-% jeweils bezogen auf die Gesamtzusammensetzung des jeweiligen Mittels erhalten.

Generell sind die kationischen aminofunktionellen Silikonpolymere sind in den erfindungsgemäßen Zusammensetzungen in Mengen von 0,01 bis 10 Gew.-%, bevorzugt in Mengen von 0,05 bis 10 Gew.% und ganz besonders bevorzugt in Mengen von 0,1 bis 7,5 Gew.-% enthalten. Die allerbesten Ergebnisse werden dabei mit Mengen von 0,1 bis 5 Gew.-% jeweils bezogen auf die Gesamtzusammensetzung des jeweiligen Mittels erhalten.

Als weitere Silikone neben den erfindungsgemäßen Dimethiconen, Dimethiconolen, Cyclomethiconen und/oder Amodimethiconen können wasserlösliche Silikone in den erfindungsgemäßen Zusammensetzungen enthalten sein.

Entsprechende hydrophile Silikone werden beispielsweise aus den Verbindungen der Formeln (Si-6) und/oder (Si-7) ausgewählt. Insbesondere bevorzugte wasserlösliche Tenside auf Silikonbasis sind ausgewählt aus der Gruppe der Dimethiconcopolyole die bevorzugt alkoxyliert, insbesondere polyethoxyliert oder polypropoxyliert sind.

Unter Dimethiconcopolyolen werden erfindungsgemäß bevorzugt Polyoxyalkylen-modifizierte Dimethylpolysiloxane der allgemeinen Formeln (Si-6) oder (Si-7) verstanden: 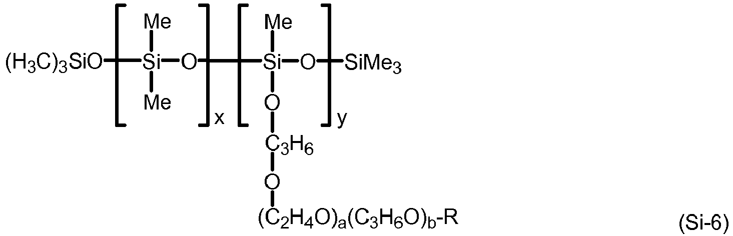 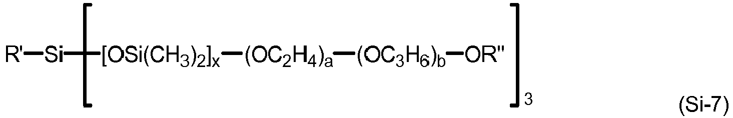 worin der Rest R steht für ein Wasserstoffatom, eine Alkylgruppe mit 1 bis 12 C-Atomen, eine Alkoxygruppe mit 1 bis 12 C-Atomen oder eine Hydroxylgruppe, die Reste R' und R" bedeuten Alkylgruppen mit 1 bis 12 C-Atomen, x steht für eine ganze Zahl von 1 bis 100, bevorzugt von 20 bis 30, y steht für eine ganze Zahl von 1 bis 20, bevorzugt von 2 bis 10 und a und b stehen für ganze Zahlen von 0 bis 50, bevorzugt von 10 bis 30.

Besonders bevorzugte Dimethiconcopolyole im Sinne der Erfindung sind beispielsweise die kommerziell unter dem Handelsnamen SILWET (Union Carbide Corporation) und DOW CORNING vertriebenen Produkte. Erfindungsgemäß besonders bevorzugte Dimethiconcopolyole sind Dow Corning 190 und Dow Corning 193.

Die Dimethiconcopolyole sind in den erfindungsgemäßen Zusammensetzungen in Mengen von 0,01 bis 10 Gew.-%, vorzugsweise 0,01 bis 8 Gew.-%, besonders bevorzugt 0,1 bis 7,5 Gew.-% und insbesondere 0,1 bis 5 Gew.-% an Dimethiconcopolyol bezogen auf die Zusammensetzung.

Die Mengen aller Silikone stehen erfindungsgemäß zur Menge aller Esteröle in einem Verhältnis von 1000 : 1 bis 50 : 1, vorzugsweise von 500 : 1 bis 50 : 1 und besonders bevorzugt von 200 : 1 bis 50 : 1 und höchst bevorzugt von 150 : 1 bis 50 : 1.

Der erfindungsgemäße Inhaltsstoff c) ist ein Derivat der Acrylsäure. Polymere, welche Acrylsäure und Acrylsäurederivate enthalten, fallen nicht unter die Definition der Derivate der Acrylsäure. Insbesondere sind unter den Derivaten von Acrylsäure die in der folgenden Formel c) beschriebenen Verbindungen zu verstehen. 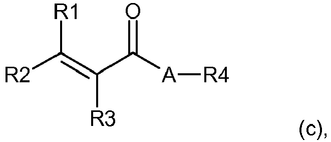 in welcher die Reste R1, R2, R3 jeweils unabhängig voneinander stehen für einen Rest ausgewählt aus Wasserstoff, einen linearen und/oder verzweigten, gesättigten und/oder ungesättigten C1 bis C30 Alkyl- oder Alkenylresten, insbesondere Methyl, Ethyl, Propyl, i-Propyl, Butyl, i-Butyl, t-Butyl, Pentyl, Amyl, i-Amyl, neo-Pentyl, Hexyl, i-Hexyl, 2-Ethylhexyl, Heptanyl, i-Heptanyl, Octanyl, i-Octanyl, Nonyl, Decyl, Lauryl, Myristyl, Aryl, Phenyl, Methoxyphenyl, Hydroxyphenyl, Methoxybenzyl, Hydroxybenzyl, Benzyl, Naphthyl oder-CN,
A steht für O oder-NR5 und
R4 sowie gegebenenfalls R5 stehen unabhängig voneinander für einen Rest ausgewählt aus Wasserstoff, einen linearen und/oder verzweigten, gesättigten und/oder ungesättigten C1 bis C30 Alkyl- oder Alkenylresten, insbesondere Methyl, Ethyl, Propyl, i-Propyl, Butyl, i-Butyl, t-Butyl, Pentyl, Amyl, i-Amyl, neo-Pentyl, Hexyl, i-Hexyl, 2-Ethylhexyl, Heptanyl, i-Heptanyl, Octanyl, i-Octanyl, Nonyl, Decyl, Lauryl, Myristyl, Aryl, Phenyl, Methoxyphenyl, Hydroxyphenyl, Methoxybenzyl, Hydroxybenzyl, Benzyl oder Naphthyl.

Bevorzugt steht A für Sauerstoff und die Verbindungen erfüllen die Formel (c1), 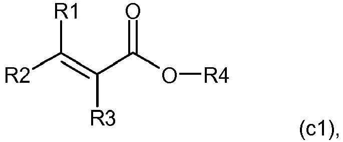 in welcher die Reste R1, R2 und R3 jeweils unabhängig voneinander stehen können für einen Rest ausgewählt aus Wasserstoff, einen linearen und/oder verzweigten, gesättigten und/oder ungesättigten C1 bis C30 Alkyl- oder Alkenylresten, insbesondere Methyl, Ethyl, Propyl, i-Propyl, Butyl, i-Butyl, t-Butyl, Pentyl, Amyl, i-Amyl, neo-Pentyl, Hexyl, i-Hexyl, 2-Ethylhexyl, Heptanyl, i-Heptanyl, Octanyl, i-Octanyl, Nonyl, Decyl, Lauryl, Myristyl, Aryl, Phenyl, Methoxyphenyl, Hydroxyphenyl, Methoxybenzyl, Hydroxybenzyl, Benzyl, Naphthyl oder -CN. R4 ist ausgewählt aus aus Wasserstoff, einen linearen und/oder verzweigten, gesättigten und/oder ungesättigten C1 bis C30 Alkyl- oder Alkenylresten, insbesondere Methyl, Ethyl, Propyl, i-Propyl, Butyl, i-Butyl, t-Butyl, Pentyl, Amyl, i-Amyl, neo-Pentyl, Hexyl, i-Hexyl, 2-Ethylhexyl, Heptanyl, i-Heptanyl, Octanyl, i-Octanyl, Nonyl, Decyl, Lauryl, Myristyl, Aryl, Phenyl, Methoxyphenyl, Hydroxyphenyl, Methoxybenzyl, Hydroxybenzyl, Benzyl oder Naphthyl.

Bevorzugte Verbindungen der Formel (c1) zeichnen sich dadurch aus, dass mindestens einer der Reste R1, R2 oder R3 steht für einen Rest ausgewählt aus Wasserstoff, Aryl, Phenyl, Methoxyphenyl, Hydroxyphenyl, Methoxybenzyl, Hydroxybenzyl, Benzyl oder Naphthyl und der Rest R4 ausgewählt ist aus Methyl, Ethyl, Propyl, i-Propyl, Butyl, i-Butyl, t-Butyl, Pentyl, Amyl, i-Amyl, neo-Pentyl, Hexyl, i-Hexyl, 2-Ethylhexyl, Heptanyl, i-Heptanyl, Octanyl, i-Octanyl, Nonyl, Decyl, Lauryl oder Myristyl. Höchst bevorzugt sind Verbindungen der Formel (c1), wenn mindestens zwei der Reste R1, R2 oder R3 für Wasserstoff stehen und der verbleibende Rest steht für einen Rest ausgewählt aus Aryl, Phenyl, Methoxyphenyl, Hydroxyphenyl, Methoxybenzyl, Hydroxybenzyl, Benzyl oder Naphthyl, bevorzugt für Phenyl oder Methoxyphenyl und der Rest R4 steht für einen Rest ausgewählt aus Methyl, Ethyl, Propyl, i-Propyl, Butyl, i-Butyl, t-Butyl, Pentyl, Amyl, i-Amyl, neo-Pentyl, Hexyl, i-Hexyl, 2-Ethylhexyl, Heptanyl, i-Heptanyl, Octanyl, i-Octanyl, Nonyl, Decyl, Lauryl oder Myristyl, bevorzugt i-Amyl oder Ethylhexyl.

Weitere höchst bevorzugte Verbindungen der Formel (c1) zeichnen sich dadurch aus, dass mindestens zwei der Reste R1, R2 oder R3 stehen für einen Rest ausgewählt aus Wasserstoff, Aryl, Phenyl, Methoxyphenyl, Hydroxyphenyl, Methoxybenzyl, Hydroxybenzyl, Benzyl oder Naphthyl und der Rest R4 ausgewählt ist aus Methyl, Ethyl, Propyl, i-Propyl, Butyl, i-Butyl, t-Butyl, Pentyl, Amyl, i-Amyl, neo-Pentyl, Hexyl, i-Hexyl, 2-Ethylhexyl, Heptanyl, i-Heptanyl, Octanyl, i-Octanyl, Nonyl, Decyl, Lauryl oder Myristyl.

Ganz besonders bevorzugte Verbindungen der Formel (c1) sind wie folgt gekennzeichnet:
R1 und R2 stehen für unabhängig voneinander für einen Rest ausgewählt aus Wasserstoff, Aryl, Phenyl, Methoxyphenyl, Hydroxyphenyl, Methoxybenzyl, Hydroxybenzyl, Benzyl oder Naphthyl, bevorzugt Phenyl oder Methoxyphenyl,
R3 steht für Wasserstoff oder -CN und R4 steht für einen Rest ausgewählt aus Methyl, Ethyl, Propyl, i-Propyl, Butyl, i-Butyl, t-Butyl, Pentyl, Amyl, i-Amyl, neo-Pentyl, Hexyl, i-Hexyl, 2-Ethylhexyl, Heptanyl, i-Heptanyl, Octanyl, i-Octanyl, Nonyl, Decyl, Lauryl oder Myristyl, bevorzugt i-Amyl oder Ethylhexyl.
Höchst bevorzugte Verbindungen der Formel (c1) sind dadurch gekennzeichnet, dass R1 = R2 ist und ausgewählt sind aus Wasserstoff, Aryl, Phenyl, Methoxyphenyl, Hydroxyphenyl, Methoxybenzyl, Hydroxybenzyl, Benzyl oder Naphthyl, bevorzugt Phenyl oder Methoxyphenyl,
R3 steht für Wasserstoff oder -CN und R4 steht für einen Rest ausgewählt aus Methyl, Ethyl, Propyl, i-Propyl, Butyl, i-Butyl, t-Butyl, Pentyl, Amyl, i-Amyl, neo-Pentyl, Hexyl, i-Hexyl, 2-Ethylhexyl, Heptanyl, i-Heptanyl, Octanyl, i-Octanyl, Nonyl, Decyl, Lauryl oder Myristyl, bevorzugt i-Amyl oder Ethylhexyl.
Am bevorzugtesten ist die Verbindung der Formel (c1) in welcher R1 = R2 = Phenyl, R3 = -CN und R4 = 2-Ethylhexyl ist.

Die erfindungsgemäßen Acrylsäurederivate der Formel (c) werden in einer Menge von 0,01 bis 5,0 Gew.-% bezogen auf die gesamte Zusammensetzung verwendet. Bevorzugt sind Mengen von 0,01 bis 3 Gew.-%, besonders bevorzugt von 0,01 bis 2,0 Gew.-% und höchst bevorzugt von 0,05 bis 2,0 Gew.-%.

Die vorliegende erfindungsgemäße Wirkstoffkombination kann als rinse-off Anwendung oder als leave-on Anwendung konfektioniert werden. Eine Anwendung als leave-on Anwendung ist bevorzugt. Innerhalb der leave-on Anwendungen ist eine Konfektionierung als Non-Aerosol Spray besonders bevorzugt.

In vielen Fällen enthalten die Mittel mindestens eine oberflächenaktive Substanz, wobei prinzipiell sowohl anionische als auch zwitterionische, ampholytische, nichtionische und kationische oberflächenaktive Substanzen geeignet sind. Die Auswahl der oberflächenaktiven Substanzen richtet sich nach der Art des Mittels. Im Falle eines Shampoos wird insbesondere mindestens ein Tensid aus der Gruppe der anionischen, der zwitterionischen oder nichtionischen oberflächenaktiven Substanzen gewählt. Bevorzugt ist hierbei, dass mindestens eine anionische und mindestens eine zwitterionische oberflächenaktive Substanz gewählt wird. Besonders bevorzugt werden diese oberflächenaktiven Substanzen dabei aus der Gruppe der besonders milden oberflächenaktiven Substanzen gewählt. In vielen Fällen hat es sich aber als vorteilhaft erwiesen, die Tenside aus anionischen, zwitterionischen oder nichtionischen Tensiden auszuwählen. Dabei beträgt das Verhältnis zwischen anionischen und zwitterionischen oberflächenaktiven Substanzen zwischen 10 : 1 und 1 : 5. Besonders bevorzugt ist das Verhältnis 5 : 1 bis 1 : 2.

Als anionische Tenside (Tanion) eignen sich in erfindungsgemäßen Zubereitungen alle für die Verwendung am menschlichen Körper geeigneten anionischen oberflächenaktiven Stoffe. Folgende anionische Tenside haben sich als mild bis besonders mild erwiesen und sind erfindungsgemäß besonders bevorzugt:
- Acyllactylate,
- Hydroxymischethersulfate,
- Ethercarbonsäuren der Formel R-O-(CH₂-CH₂0)ₓ-CH₂-COOH, in der R eine lineare Alkylgruppe mit 8 bis 30 C-Atomen und x = 0 oder 1 bis 16 ist und deren Salze,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen.
- Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel, R¹(OCH₂CH₂)ₙ-O-(PO-OX)-OR²,
   in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- Monoglyceridsulfate und Monoglyceridethersulfate der Formel ROC-(OCH₂CH₂)x-OCH₂-[CHO(CH₂CH₂O)_{y}H]-CH₂O(CH₂CH₂O)_{z}-SO₃X,
   in der RCO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate eingesetzt, in der RCO für einen linearen Acylrest mit 8 bis 18 Kohlenstoffatomen steht,
- Amidethercarbonsäuren, R¹-CO-NR²-CH₂CH₂-O-(CH₂CH₂O)ₙCH₂COOM, mit R¹ als geradkettigen oder verzweigten Alkyl- oder Alkenylrest mit einer Zahl an Kohlenstoffatomen in der Kette von 2 bis 30, n steht für eine ganze Zahl von 1 bis 20 und R² steht für Wasserstoff, einen Methyl-, Ethyl-, Propyl-, Isopropyl-, n-Butyl-, t-Butyl- oder iso-Butylrest und M steht für Wasserstoff oder ein Metall wie Alkalimetall, insbesondere Natrium, Kalium, Lithium, Erdalkalimetall, insbesondere Magnesium, Calcium, Zink, oder ein Ammoniumion, wie ⁺NR³R⁴R⁵R⁶, mit R³ bis R⁶ unabhängig voneinander stehend für Wasserstoff oder einen C1 bis C4 - Kohlenwasserstoffrest. Derartige Produkte sind beispielsweise von der Firma Chem-Y unter der Produktbezeichnung Akypo^{®} erhältlich.
- Kondensationsprodukte aus einem wasserlöslichen Salz eines wasserlöslichen Eiweißhydrolysates mit einem geeigneten Fettsäurederivat, beispielsweise einem C8 - C30 Fettsäurehalogenid. Solche Produkte sind unter dem Warenzeichen Lamepon^{®}, Maypon^{®}, Gluadin^{®}, Hostapon^{®} KCG oder Amisoft^{®} seit langem im Handel erhältlich,
- Acylglutamate und
- Acylaspartate.

Weiterhin ist es im Falle von milden anionischen Tensiden mit Polyglycolethereinheiten bevorzugt, dass die Zahl der Glykolethergruppen 1 bis 20 beträgt, bevorzugt 2 bis 15, besonders bevorzugt 2 bis 12. Besonders milde anionische Tenside mit Polyglykolethergruppen ohne eingeschränkte Homologenverteilung können beispielsweise auch erhalten werden, wenn einerseits die Zahl der Polyglykolethergruppen 4 bis 12 beträgt und als Gegenion Zn- oder Mg-ionen gewählt werden. Beispiel hierfür ist das Handelsprodukt Texapon^{®} ASV.

Als zwitterionische Tenside (Tzwitter) werden solche oberflächenaktiven Verbindungen bezeichnet, die im Molekül mindestens eine quartäre Ammoniumgruppe und mindestens eine -COO⁽⁻⁾ - oder - SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden (Tampho) werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Aminopropionate, Aminoglycinate, Imidazoliniumbetaine und Sulfobetaine.

Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside sind beispielsweise
- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol^{®} - Typen (Cognis),
- alkoxilierte Triglyceride,
- alkoxilierte Fettsäurealkylester der Formel (Tnio-1)

   R¹CO-(OCH₂CHR²)_{w}OR³ (Tnio-1)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (Tnio-2)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht.
- Zuckertenside vom Typ der Fettsäure-N-alkylpolyhydroxyalkylamide, ein nichtionisches Tensid der Formel (Tnio-3),

   R-⁵CO-NR⁶-[Z] (Tnio-3)

   in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Eine ganz besonders bevorzugte Ausführungsform enthält mindestens ein kationisches Tensid. Kationische Tenside (Tkat) leiten sich im allgemeinen von Ammoniumionen ab und besitzen eine Struktur (NR¹R²R³R⁴)⁺ A⁻ (Tkat1)
mit einem entsprechend negativ geladenen Gegenion A.

Bei allen erfindungsgemäßen kationischen Tensiden ist das Anion A ausgewählt aus den physiologisch verträglichen Anionen. Beispielhaft für das Anion seien die Halogenide, Fluorid, Chlorid, Bromid, Iodid, Phosphat, Dihydrogenphosphat, Hydogenphosphat, Hydrogensulfat, Sulfat (Methosulfat) der allgemeinen Formel RSO₃⁻ worin R die Bedeutung von gesättigtem oder ungesättigtem Alkylresten mit 1 bis 4 Kohlenstoffatomen hat, insbesondere Methosulfat und Ethosulfat, oder anionische Reste organischer Säuren wie Maleat, Fumarat, Oxalat, Tartrat, Citrat, Lactat oder Acetat, genannt. Besonders bevorzugte kationische Tenside haben als Anion das Chlorid oder Methosulfat mit einer Methyl-, oder Ethylgruppe als Rest R.

Derartige kationische Ammoniumverbindungen der Formel (Tkat1) sind dem Fachmann bestens bekannt. Die Reste R1 bis R4 können jeweils unabhängig voneinander geradkettige, verzweigte, cyclische, aromatische gesättigte oder ungesättigte Alkyl- und/oder Alkenylketten mit mindestens 1 bis 30 Kohlenstoffatomen, Wasserstoff, -OH oder Hydroxyethyl- bedeuten.

Erfindungsgemäß bevorzugt sind kationische Strukturen, in welchen die Reste R1, R2 und R3 jeweils eine Methylgruppe bedeuten und R4 einen gesättigten, verzweigten oder unverzweigten Alkylrest mit einer Kettenlänge von 16 bis 24 Kohlenstoffatomen darstellt und weiterhin der Rest R4 einen gesättigten, verzweigten oder unverzweigten Alkylrest, ganz besonders bevorzugt unverzweigten Alkylrest mit einer Kettenlänge von 18 bis 24, höchst bevorzugt mit einer Kettenlänge von 22 bis 24 Kohlenstoffatomen darstellt. Bevorzugte quaternäre Ammoniumverbindungen der Struktur (Tkat1) sind Ammoniumhalogenide, insbesondere Chloride und Bromide, wie Alkyltrimethylammoniumchloride, Dialkyldimethylammoniumchloride und Trialkylmethylammoniumchloride, z.B. Distearyldimethylammoniumchlorid, Lauryldimethylammoniumchlorid, Lauryldimethylbenzylammoniumchlorid und Tricetylmethylammoniumchlorid Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat. Bevorzugt sind Cetyltrimethylammonium- und Behenyltrimethylammoniumsalze. Besonders bevorzugt sind letztere in Form der Methosulfate und der Bromide. Am bevorzugtesten sind Cetyltrimethylammoniummethosulfat und Behenyltrimethylammoniummethosulfat und höchst bevorzugt ist Behenyltrimethylammoniummethosulfat.

Weiterhin können in einer Ausführungsform Esterquats gemäß der Formel (Tkat1-2) verwendet werden.

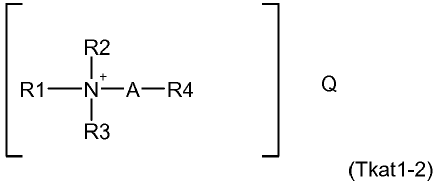

Hierin sind die Reste R1, R2 und R3 jeweils unabhängig voneinander und können gleich oder verschieden sein. Die Reste R1, R2 und R3 bedeuten:
- ein verzweigter oder unverzweigter Alkylrest mit 1 bis 4 Kohlenstoffatomen, welcher mindestens eine Hydroxylgruppe enthalten kann, beispielsweise Methyl, Ethyl, Propyl, Isopropyl, Butyl, Isobutyl, t-Butyl, Hydroxyethyl, Hydroxymethyl, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- ein Aryl oder Alkarylrest, beispielsweise Phenyl oder Benzyl,
- den Rest (- A - R4), mit der Maßgabe, daß höchstens 2 der Reste R1, R2 oder R3 für diesen Rest stehen können:

Der Rest -(A - R4) ist mindestens 1 bis 3 mal enthalten.

Hierin steht A für:
1) -(CH2)n- mit n = 1 bis 20, vorzugsweise n = 1 bis 10 und besonders bevorzugt n = 1 - 5, oder
2) -(CH2-CHR5-O)n- mit n = 1 bis 200, vorzugsweise 1 bis 100, besonders bevorzugt 1 bis 50, und besonders bevorzugt 1 bis 20 mit R5 in der Bedeutung von Wasserstoff, Methyl oder Ethyl,
und R4 steht für:
1) R6-O-CO-, worin R6 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder
2) R7-CO-, worin R7 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann,
und Q steht für ein physiologisch verträgliches organisches oder anorganisches Anion, welches wie zuvor unter Formel (Tkat1) beschrieben wurde. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Rewoquat^{®}, Stepantex^{®}, Dehyquart^{®} und Armocare^{®} vertrieben. Die Produkte Armocare^{®} VGH-70, ein N,N-Bis(2-Palmitoyloxyethyl)dimethylammonium-chlorid, sowie Dehyquart^{®} F-75, Dehyquart^{®} C-4046, Dehyquart^{®} L80, Dehyquart^{®} F-30, Dehyquart^{®} AU-35, Rewoquat^{®} WE18, Rewoquat^{®} WE38 DPG und Stepantex^{®} GS 90 sind Beispiele für solche Esterquats.

Weitere erfindungsgemäß besonders bevorzugte Verbindungen der Formel (Tkat1-2) zählen zur Formel (Tkat1-2.1 den kationischen Betainestern.

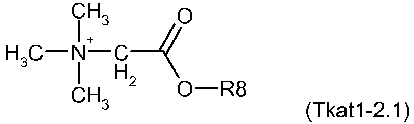

R8 entspricht in seiner Bedeutung R7.

Weitere bevorzugte kationische Tenside sind kationische Tenside der Formel (Tkat-2).

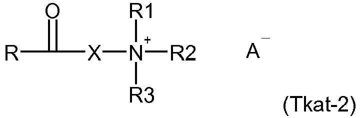

R steht hierin für einen substituierten oder unsubstituierten, verzweigten oder geradkettigen Alkyl- oder Alkenylrest mit 11 bis 35 Kohlenstoffatomen in der Kette,
X steht für - O - oder - NR⁵ -,

R¹ steht für eine Alkylengruppe mit 2 bis 6 C - Atomen, welche nicht substituiert oder substituiert sein kann, wobei im Falle einer Substitution die Substitution mit einer -OH - oder -NH- Gruppe bevorzugt ist,

R² und R³ jeweils unabhängig voneinander stehen für eine Alkyl oder Hydroxyalkylgruppe mit 1 bis zu 6 C - Atomen in der Kette, wobei die Kette geradlinig oder verzweigt sein kann. Beispiele für erfindungsgemäße Reste sind Methyl, Ethyl, Propyl, iso-Propyl, Butyl, iso-Butyl, tert-Butyl, Pentyl, iso-Pentyl, neo-Pentyl, Hexyl, iso-Hexyl, Hydroxyalkyl, Dihydroxyalkyl, Hydroxyethyl, Hydroxypropyl, Dihydroxypropyl, Hydroxybutyl, Dihydroxybutyl, Trihydroxybutyl, Trihydroxypropyl, Dihydroxyethyl,

R5 steht für Wasserstoff oder einen C1 bis C6 geradkettigen oder verzweigten, Alkyl- oder Alkenylrest, welcher auch durch eine Hydroxygruppe substituiert sein kann, besonders Methyl, Ethyl, Propyl, iso-Propyl, Butyl, iso-Butyl, tert-Butyl, Pentyl, iso-Pentyl, neo-Pentyl, Hexyl, iso-Hexyl, Hydroxyethyl, Hydroxypropyl, Dihydroxypropyl, Hydroxybutyl, Dihydroxybutyl, Trihydroxybutyl, Trihydroxypropyl, Dihydroxyethyl und

A⁻ bedeutet ein Anion wie bereits bei der Formel (Tkat1) beschrieben.

Innerhalb dieser Strukturklasse werden bevorzugt die Verbindungen einer der folgenden Strukturen verwendet:

CH₃(CH₂)₂₀CONH(CH₂)₃ - N⁺(CH₃)₂-CH₂CH₃ A⁻ (Tkat-3)

CH₃(CH₂)₂₀CONH(CH₂)₃ - N⁺(CH₃)₂-CH₂(CHOH)CH₂OH A⁻ (Tkat-4)

CH₃(CH2)₂₀COOCH₂CHOHCH₂ - N⁺(CH₃)₃ A⁻ (Tkat-5)

CH₃(CH₂)₂₀CONH(CH₂)₃ - N⁺(CH₃)₂-CH₂CH₂OH A⁻ (Tkat-6)

Beispiele für derartige Handelsprodukte sind Schercoquat BAS, Lexquat AMG-BEO, Akypoquat 131 oder Incroquat Behenyl HE.

In einer besonders bevorzugten Ausführungsform der Erfindung enthalten die erfindungsgemäßen Mittel weiterhin mindestens ein Amin und/oder kationisiertes Amin, insbesondere ein Amidoamin und/oder ein kationisiertes Amidoamin mit den folgenden Strukturformeln:

R¹ - NH - (CH₂)ₙ - NR²R³ (Tkat7)

und/oder

R¹ - NH - (CH₂)ₙ - NR²R³R⁴ (Tkat8)

worin R1 ein Acyl- oder Alkylrest mit 6 bis 30 C-Atomen, welche verzweigt oder unverzweigt, gesättigt oder ungesättigt sein können, und wobei der Acylrest und/oder der Alkylrest mindestens eine OH-Gruppe enthalten können, und
R2, R3 und R4 jeweils unabhängig voneinander Wasserstoff oder ein Alkylrest mit 1 bis 4 C-Atomen, welcher gleich oder verschieden, gesättigt oder ungesättigt sein kann, und
n eine ganze Zahl zwischen 1 und 10 bedeuten.

Das Anion ist wiederum ausgewählt aus den physiologisch verträglichen Anionen wie bereits bei der Formel (Tkat1) beschreiben.

Bevorzugt wird eine Zusammensetzung, in welcher das Amin und/oder das quaternisierte Amin gemäß allgemeiner Formeln (Tkat7) und/oder (Tkat8) ein Amidoamin und/oder ein quaternisiertes Amidoamin ist, worin R1 ein verzweigter oder unverzweigter, gesättigter oder ungesättigter Acylrest mit 6 bis 30 C-Atomen, welcher mindestens eine OH-Gruppe enthalten kann, bedeutet. Bevorzugt ist hierbei ein Fettsäurerest aus Ölen und Wachsen, insbesondere aus natürlichen Ölen und Wachsen, ist. Als Beispiele hierfür kommen Lanolin, Bienen- oder Candellilawachse in Betracht. Bevorzugt sind auch solche Amidoamine und/oder quaternisierte Amidoamine, in denen R2, R3 und/oder R4 in Formeln (Tkat7) und/oder (Tkat8) ein Rest gemäß der allgemeinen Formel CH₂CH₂OR5 bedeuten, worin R5 die Bedeutung von Alkylresten mit 1 bis 4 Kohlenstoffatomen, Hydroxyethyl oder Wasserstoff haben kann. Die bevorzugte Größe von n in den allgemeinen Formeln (Tkat7) und/oder (Tkat8) ist eine ganze Zahl zwischen 2 und 5.

Weiterhin bevorzugt sind Amidoamine und/oder quaternisierte Amidoamine der allgemeinen Formeln (Tkat7) und/oder (Tkat8), in denen das Anion ein Halogenidion oder eine Verbindung der allgemeinen Formel RSO₃ ist, worin R die Bedeutung von gesättigtem oder ungesättigtem Alkylresten mit 1 bis 4 Kohlenstoffatomen hat.

Die Alkylamidoamine können sowohl als solche vorliegen und durch Protonierung in entsprechend saurer Lösung in eine quaternäre Verbindung in der Zusammensetzung überführt werden. Erfindungsgemäß bevorzugt sind die kationischen Alkylamidoamine.

Als erfindungsgemäß zu verwendende Amidoamine, welche gegebenenfalls quaternisiert sein können, kommen beispielsweise in Betracht als Amidoamine: Witcamine 100 (Witco, INCI-Bezeichnung: Cocamidopropyl Dimethylamine), Incromine BB (Croda, INCI-Bezeichnung: Behenamidopropyl Dimethylamine), Mackine 401 (Mclntyre, INCI-Bezeichnung: Isostearylamidopropyl Dimethylamine) und andere Mackine-Typen, Adogen S18V (Witco, INCI-Bezeichnung: Stearylamidopropyl Dimethylamine), und als permanent kationische Aminoamine: Rewoquat RTM 50 (Witco Surfactants GmbH, INCI-Bezeichnung: Ricinoleamidopropyltrimonium Methosulfate), Empigen CSC (Albright&Wilson, INCI-Bezeichnung: Cocamidopropyltrimonium Chlorid), Swanol Lanoquat DES-50 (Nikko, INCI-Bezeichnung: Quatemium-33), Rewoquat UTM 50 (Witco Surfactants GmbH, Undecyleneamidopropyltrimonium Methosulfate).

Schließlich können als kationische Tenside quartäre Imidazolinverbindungen, d.h. Verbindungen, die einen positiv geladenen Imidazolinring aufweisen, verwendet werden. Die im Folgenden dargestellte Formel (Tkat9) zeigt die Struktur dieser Verbindungen.

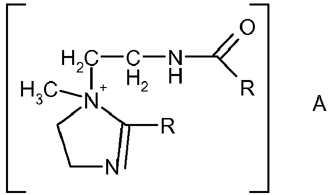

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (Tkat9) enthalten für R jeweils den gleichen Kohlenwasserstoffrest. Die Reste R stehen unabhängig voneinander jeweils für einen gesättigten oder ungesättigten, linearen oder verzweigten Kohlenwasserstoffrest mit einer Kettenlänge von 8 bis 18 Kohlenstoffatomen. Die bevorzugten Verbindungen der Formel I enthalten für R jeweils den gleichen Kohlenwasserstoffrest. Die Kettenlänge der Reste R beträgt bevorzugt 12 bis 18 Kohlenstoffatome. Besonders bevorzugt sind Verbindungen mit einer Kettenlänge von mindestens 16 Kohlenstoffatomen und ganz besonders bevorzugt mit 18 Kohlenstoffatomen. Die Kettenlänge der Reste R beträgt höchst bevorzugt mindestens 20 Kohlenstoffatome. Am bevorzugtesten sind Verbindungen mit einer Kettenlänge von mindestens 21 Kohlenstoffatomen. Bevorzugte Reste R sind Cetyl, Oleyl, Palmityl, Stearyl und Behenyl. A bedeutet ein physiologisch verträgliches Anion, wie bereits bei den kationischen Verbindungen der Formel (Tkat1) beschrieben. Besonders erfindungsgemäße Beispiele sind unter den INCII - Bezeichnungen Quaternium-27, Quaternium-72 und Quaternium-83 erhältlich. Ein höchst bevorzugtes Handelsprodukt dieser Kettenlänge ist beispielsweise unter der Bezeichnung Quaternium-91 bekannt.

Selbstverständlich umfasst die erfindungsgemäße Lehre auch, dass mindestens zwei kationische Tenside verwendet werden. In diesem Falle ist es bevorzugt, wenn die kationischen Tenside aus zwei unterschiedlichen Strukturklassen gewählt werden.

Die kationischen Tenside sind in den erfindungsgemäßen Zusammensetzungen in Mengen von 0,01 bis 20 Gew.%, bevorzugt in Mengen von 0,01 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

Kationische, zwitterionische und/oder amphotere Tenside sowie deren Mischungen können erfindungsgemäß bevorzugt sein.

Die Tenside (T) werden in Mengen von 0,05 - 45 Gew.%, bevorzugt 0,1 - 30 Gew.% und ganz besonders bevorzugt von 0,5 - 25 Gew.%, bezogen auf das gesamte erfindungsgemäß verwendete Mittel, eingesetzt.

Erfindungsgemäß verwendbare Emulgatoren sind beispielsweise
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate wie bereits bei den Tensiden beschrieben,
- Monoglyceridsulfate und Monoglyceridethersulfate wie bereits bei den Tensiden beschrieben,
- Amidethercarbonsäuren wie sie im Kapitel über Tenside beschrieben sind,
- Kondensationsprodukte aus einem wasserlöslichen Salz eines wasserlöslichen Eiweißhydrolysat-Fettsäure-Kondensationsproduktes,
- Zwitterionische Tenside (Tzwitter)),
- Ampholytische Tenside (Tampho),
- Zuckertenside vom Typ der Alkyl- oder Alkenyloligoglykoside,
- Zuckertenside vom Typ der Fettsäure-N-alkylpolyhydroxyalkylamide,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol^{®} - Typen (Cognis),
- Aminoxide,
- Hydroxymischether, beispielsweise der Formel R¹O[CH₂CH(CH₃)O]ₓ(CH₂CHR²O)_{y}[CH₂CH(OH)R³]_{z} mit R¹ stehend für einen linearen oder verzweigten, gesättigten oder ungesättigten Alkyl- und/oder Alkenylrest mit 2 bis 30 C - Atomen, R² stehend für Wasserstoff, einen Methyl-, Ethyl-, Propyl- oder iso-Propylrest, R³ stehend für einen linearen oder verzweigten Alkylrest mit 2 bis 30 C - Atomen, x stehend für 0 oder eine Zahl von 1 bis 20, Y für eine Zahl von 1 bis 30 und z stehend für die Zahl 1, 2, 3 , 4 oder 5.
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),
- Esterquats,
- Alkylamidoamine und quaternierte Alkylamidoamine.
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine,
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),

In den erfindungsgemäßen Mitteln werden ganz besonders kationische und/oder amphotere und/oder zwitterionische Polymere als weitere Inhaltsstoffe verwendet.

Im Folgenden werden einige Beispiele von besonders bevorzugten Polymeren beschrieben.

Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium- 11).

Von den kationischen Polymeren, die in dem erfindungsgemäßen Mittel enthalten sein können, ist zum Beispiel Vinylpyrrolidon/Dimethylaminoethylmethacrylatmethosulfat Copolymer, das unter den Handelsbezeichnungen Gafquat^{®} 755 N und Gafquat^{®} 734 von der Firma Gaf Co., USA vertrieben wird und von denen das Gafquat^{®} 734 besonders bevorzugt ist, geeignet. Weitere kationische Polymere sind beispielsweise das von der Firma BASF, Deutschland unter dem Handelsnamen Luviquat^{®} HM 550 vertriebene Copolymer aus Polyvinylpyrrolidon und Imidazoliminmethochlorid, das von der Firma Calgon/USA unter dem Handelsnamen Merquat^{®} Plus 3300 vertriebene Terpolymer aus Dimethyldiallylammoniumchlorid, Natriumacrylat und Acrylamid und das von der Firma ISP unter dem Handelsnamen Gafquat^{®} HS 100 vertriebene Vinylpyrrolidon/Methacryl-amidopropyltrimethylammoniumchlorid Copolymer.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (3V Sigma) im Handel erhältlich. Weitere Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate.

Eine kationische Cellulose wird unter der Bezeichnung Polymer JR^{®} 400 von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Weitere Handelsprodukte sind die Verbindungen Celquat^{®} H 100, Celquat^{®} und L 200. Die genannten Handelsprodukte sind bevorzugte kationische Cellulosen.

Geeignete kationische Guarderivate werden unter der Handelsbezeichnung Jaguar^{®} vertrieben und haben die INCI-Bezeichnung Guar Hydroxypropyltrimonium Chloride. Weiterhin werden besonders geeignete kationische Guarderivate auch von der Fa. Hercules unter der Bezeichnung N-Hance^{®} im Handel. Weitere kationische Guarderivate werden von der Fa. Cognis unter der Bezeichnung Cosmedia^{®} vertrieben. Ein bevorzugtes kationisches Guarderivat ist das Handelsprodukt AquaCat^{®} der Fa. Hercules. Bei diesem Rohstoff handelt es sich um ein bereits vorgelöstes kationisches Guarderivat.

Ein geeignetes Chitosan wird beispielsweise von der Firma Kyowa Oil& Fat, Japan, unter dem Handelsnamen Flonac^{®} vertrieben. Ein bevorzugtes Chitosansalz ist Chitosoniumpyrrolidoncarboxylat, welches beispielsweise unter der Bezeichnung Kytamer PC von der Firma Amerchol, USA, vertrieben wird. Weitere Chitosanderivate sind unter den Handelsbezeichnungen Hydagen^{®} CMF, Hydagen^{®} HCMF und Chitolam^{®} NB/101 im Handel frei verfügbar.

Weitere bevorzugte kationische Polymere sind beispielsweise
- kationische Alkylpolyglycoside,
- kationisierter Honig, beispielsweise das Handelsprodukt Honeyquat^{®} 50,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium-2, Polyquaternium-17, Polyquaternium-18 und Polyquaternium-27 bekannten Polymeren mit quartären Stickstoffatomen in der Polymerhauptkette,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind. Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt.

Die kationischen Polymere sind in den erfindungsgemäßen Zusammensetzungen bevorzugt in Mengen von 0,05 bis 10 Gew.-%, bezogen auf das gesamte Mittel, enthalten. Mengen von 0,1 bis 5 Gew.-% sind besonders bevorzugt.

Amphotere Polymere sind ebenso wie die kationischen Polymere ganz besonders bevorzugte Polymere. Besonders bevorzugte amphotere Polymere sind Copolymere, aus Diallyldimethylammoniumchlorid und Acrylsäure. Diese Copolymerisate werden unter der INCI-Bezeichnung Polyquaternium-22 unter anderem mit dem Handelsnamen Merquat^{®} 280 (Nalco) vertrieben. Erfindungsgemäß ganz besonders bevorzugt verwendete amphotere Polymere sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat^{®} Plus 3330 (Nalco) vertrieben.

Die amphoteren Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Bei den anionischen Polymeren handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann. Besonders bevorzugt ist das Homopolymer der 2-Acrylamido-2-methylpropansulfon-säure, das beispielsweise unter der Bezeichnung Rheothik^{®}11-80 im Handel erhältlich ist.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaflin) und einen nichtionogenen Emulgator (Laureth-7) enthält, hat sich im Rahmen der erfindungsgemäßen Lehre als besonders vorteilhaft erwiesen. Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Die anionischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol^{®} (BASF) vertrieben werden.

Die nichtionischen Polymere sind in den erfindungsgemäßen Zusammensetzungen bevorzugt in Mengen von 0,05 bis 10 Gew.-%, bezogen auf das gesamte Mittel, enthalten. Mengen von 0,1 bis 5 Gew.-% sind besonders bevorzugt.

Die Polymere (P) sind in den erfindungsgemäß verwendeten Zusammensetzungen bevorzugt in Mengen von 0,01 bis 30 Gew.-%, bezogen auf die gesamte Zusammensetzung, enthalten. Mengen von 0,01 bis 25, insbesondere von 0,01 bis 15 Gew.-%, sind besonders bevorzugt.

Mit besonderem Vorzug enthalten die erfindungsgemäßen Zusammensetzungen Fettstoffe (Fat) als weiteren Wirkstoff. Unter Fettstoffen (Fat) sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Als Fettsäuren (Fatac) können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1 - 5 Gew.% sein können.

Als Fettalkohole (Fatal) können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole können ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®}C14, Lorol^{®}C18, Lorol^{®}C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 -20 Gew.-% eingesetzt.

Als natürliche oder synthetische Wachse (Fatwax) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Die Gesamtmenge an Öl- und Fettkomponenten in den erfindungsgemäßen Mitteln beträgt üblicherweise 0,5 - 75 Gew.-%, bezogen auf das gesamte Mittel. Mengen von 0,5 - 35 Gew.-% sind erfindungsgemäß bevorzugt.

Ein weiterer erfindungsgemäßer synergistischer Wirkstoff in den erfindungsgemäßen Zusammensetzungen mit dem erfindungsgemäßen Wirkstoffkomplex sind Proteinhydrolysate und/oder dessen Derivate.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Weiterhin sind erfindungsgemäß bevorzugte pflanzliche Proteinhydrolysaten wie beispielsweise Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Weitere erfindungsgemäß bevorzugte Proteinhydrolysate sind maritimen Ursprunges. Hierzu zählen beispielsweise Kollagenhydrolysate von Fischen oder Algen sowie Proteinhydrolysate von Muscheln bzw. Perlenhydrolysate. Beispiele für erfindungsgemäße Perlenextrakte sind die Handelsprodukte Pearl Protein Extract BG^{®} oder Crodarom^{®} Pearl.

Die Proteinhydrolysate (P) sind in den Zusammensetzungen in Konzentrationen von 0,001 Gew.% bis zu 20 Gew.%, vorzugsweise von 0,05 Gew.% bis zu 15 Gew.% und ganz besonders bevorzugt in Mengen von 0,05 Gew.% bis zu 5 Gew.% enthalten.

Die Wirkung der erfindungsgemäßen Zusammensetzungen kann weiterhin durch eine 2-Pyrrolidinon-5-carbonsäure und deren Derivate (J) gesteigert werden. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Eine weitere bevorzugte Gruppe von Inhaltsstoffen der erfindungsgemäßen Zusammensetzungen mit dem erfindungsgemäßen Wirkstoffkomplex sind Vitamine, Provitamine oder Vitaminvorstufen. Vitamine, Pro-Vitamine und Vitaminvorstufen sind dabei besonders bevorzugt, die den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Zusammensetzungen Vitamine, Provitamine und Vitaminvorstufen aus den Gruppen A, B, E und H. Panthenol, Pantolacton, Pyridoxin und seine Derivate sowie Nicotinsäureamid und Biotin sind besonders bevorzugt.

Schließlich ergeben sich durch die Verwendung von Pflanzenextrakten (L) in den erfindungsgemäßen Zusammensetzungen weitere Vorteile. Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Baldrian, Wiesenschaumkraut, Quendel, Schafgarbe, Thymian, Melisse, Hauhechel, Huflattich, Eibisch, Meristem, Ginseng, Kaffee, Kakao, Moringa und Ingwerwurzel bevorzugt. Erfindungsgemäß kann es höchst bevorzugt sein, wenn als Pflanzenextrakte sogenannte ayurvedische Pflanzenextrakte verwendet werden. Zu den traditionellen ayurvedischen Pflanzen zählen Aegle Marmelos (Bilwa), Cyperus Rotundus (Nagar Motha), Emblica Officinalis (Amalki), Morida Citrifolia (Ashyuka), Tinospora Cordifolia (Guduchi), Santalum album, (Chandana), Crocus Sativus (Kumkuma), Cinnamonum Zeylanicum und Nelumbo Nucifera (Kamala).

Als weiteren wesentlichen Inhaltsstoff können die erfindungsgemäßen Mittel Purin und/oder Derivat(e) des Purins enthalten. Bevorzugte erfindungsgemäße Zusammensetzungen enthalten Purin und/oder Purinderivate in engeren Mengenbereichen. Hier sind erfindungsgemäß bevorzugte kosmetische Mittel dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten.

Unter Purin, den Purinen und den Purinderivaten sind erfindungsgemäß folgende Verbindungen bevorzugt: Purin, Adenin, Guanin, Harnsäure, Hypoxanthin, 6-Purinthiol, 6-Thioguanin, Xanthin, Coffein, Theobromin und Theophyllin. In haarkosmetischen Formulierungen hat sich insbesondere Coffein bewährt, das beispielsweise in Shampoos, Conditionern, Haarwässern und/oder Lotionen vorzugsweise in Mengen von 0,005 bis 0,25 Gew.-%, weiter bevorzugt von 0,01 bis 0,1 Gew.-% und insbesondere von 0,01 bis 0,05 Gew.-% (jeweils bezogen auf die Zusammensetzung) eingesetzt werden kann.

Ein weiterer bevorzugter Wirkstoff zur zusätzlichen Verwendung in den erfindungsgemäßen Mitteln ist Taurin und/oder ein Derivat des Taurines. Unter Taurin wird ausschließlich 2-Aminoethansulfonsäure und sowie explizit genannte Derivate des Taurines verstanden. Unter den Derivaten des Taurines werden N-Monomethyltaurin und N,N-Dimethyltaurin verstanden. Als weitere Taurinderivate werden auch Taurine verstanden, welche als Stoffwechselprodukte im pflanzlichen und/oder tierischen und/oder marinen Organismen natürlicherweise vorkommen. Hierzu zählen beispielsweise, wenn auch nicht bevorzugt, Abbauprodukte des Cysteines, insbesondere die Cysteinsulfinsäure. Weitere Taurinderivate im Sinne der vorliegenden Erfindung sind die Taurocholsäure und Hypotaurin.

Besonders bevorzugte sind erfindungsgemäße Mittel, die - bezogen auf ihr Gewicht - 0,0001 bis 10,0 Gew.-%, vorzugsweise 0,0005 bis 5,0 Gew.-%, besonders bevorzugt 0,001 bis 2,0 Gew.-% und insbesondere 0,001 bis 1,0 Gew.-% Taurin und/oder eines Derivates des Taurines enthalten. Zusätzlich kann es sich als vorteilhaft erweisen, wenn in den erfindungsgemäßen Zusammensetzungen Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Eine besonders bevorzugte Gruppe von Quellmitteln können Hydantoine sein. Erfindungsgemäße Zusammensetzungen enthalten bevorzugt 0,01 bis 5 Gew.-% Hydantoin bzw. mindestens eines Hydatoinderivates. Besonders bevorzugt werden erfindungsgemäß Hydantoinderivate eingesetzt, wobei 5-Ureidohydantoin besonders bevorzugt ist. Unabhängig davon, ob Hydantoin oder Hydantoinderivat(e) eingesetzt wird/werden, sind Einsatzmengen von 0,02 bis 2,5 Gew.-% ganz besonders bevorzugt, von 0,05 bis 1,5 Gew.-%, weiter bevorzugt 0,075 bis 1 Gew.-% und insbesondere 0,1 bis 0,25 Gew.-% -jeweils bezogen auf das gesamte Mittel - höchst bevorzugt. Die erfindungsgemäße Verwendung von Polyhydroxyverbindungen als Wirkstoff mit den anderen erfindungsgemäßen Komponenten kann besonders bevorzugt sein. Unter Polyhydroxyverbindungen sind organische Verbindungen mit mindestens zwei Hydroxygruppen zu verstehen. Insbesondere sind im Sinne der vorliegenden Erfindung hierunter zu verstehen:
- Polyole mit mindestens zwei Hydroxygruppen, wie beispielsweise Trimethylolpropan,
- Kohlenhydrate, Zuckeralkohole und Zucker sowie deren Salze,
- insbesondere Monosaccharide, Disaccharide, Trisaccharide und Oligosaccharide, wobei diese auch in Form von Aldosen, Ketosen und/oder Lactosen vorliegen können,
- Aminodesoxyzucker, Desoxyzucker, Thiozucker, wobei diese auch in Form von Aldosen, Ketosen und/oder Lactosen vorliegen können,

Ganz besonders bevorzugt sind hierunter Monosaccharide mit 3 bis 8 C - Atomen, wie beispielsweise Triosen, Tetrosen, Pentosen, Hexosen, Heptosen und Octosen, wobei diese auch in Form von Aldosen, Ketosen und/oder Lactosen vorliegen können.

Weiterhin sind bevorzugt Oligosaccharide mit bis zu 50 Monomereinheiten. Beispielhaft für die erfindungsgemäßen Polyole seien erwähnt Sorbit, Inosit, Mannit, Tetrite, Pentite, Hexite, Threit, Erythrit, Adonit, Arabit, Xylit, Dulcit, Erythrose, Threose, Arabinose, Ribose, Xylose, Lyxose, Glucose, Galactose, Mannose, Allose, Altrose, Gulose, Idose, Talose, Fructose, Sorbose, Psicose, Tegatose, Desoxyribose, Glucosamin, Galaktosamin, Rhamnose, Digitoxose, Thioglucose, Saccharose, Lactose, Trehalose, Maltose, Cellobiose, Melibiose, Gestiobiose, Rutinose, Raffinose sowie Cellotriose.

Bevorzugte Polyhydroxyverbindungen sind Sorbit, Inosit, Mannit, Threit, Erythreit, Erythrose, Threose, Arabinose, Ribose, Xylose, Glucose, Galactose, Mannose, Allose, Fructose, Sorbose, Desoxyribose, Glucosamin, Galaktosamin, Saccharose, Lactose, Trehalose, Maltose und Cellobiose. Besonders bevorzugt werden Glucose, Galactose, Mannose, Fructose, Desoxyribose, Glucosamin, Saccharose, Lactose, Maltose und Cellobiose verwendet. Ganz besonders bevorzugt ist jedoch die Verwendung von Glucose, Galactose, Mannose, Fructose, Saccharose, Lactose, Maltose oder Cellobiose

Unter den Polyhydroxyverbindungen mit 3 OH-Gruppen hat das Glycerin eine herausragende Bedeutung.

Unabhängig vom Typ der eingesetzten Polyhydroxyverbindung mit mindestens 2 OH-Gruppen sind erfindungsgemäße Mittel bevorzugt, die, bezogen auf das Gewicht des Mittels, 0,01 bis 5 Gew.%, vorzugsweise 0,05 bis 4 Gew.%, besonders bevorzugt 0,05 bis 3,5 Gew.% und insbesondere 0,1 bis 2,5 Gew.% Polyhydroxyverbindung(en) enthalten.

Ganz besonders bevorzugte Polyole der vorliegenden Erfindung sind Polyole mit 2 bis 12 C-Atomen im Molekülgerüst. Diese Polyole können geradkettig, verzweigt, cyclisch und/oder ungesättigt sein. Die Hydroxygruppen sind dabei ganz besonders bevorzugt endständig benachbart oder endständig durch den Rest der Kette voneinander getrennt. Als Beispiele für diese Polyole seien genannt: Glykol, Polyethylenglykol bis zu einem Molgewicht bis zu 1000 Dalton, Neopentylglykol, Partialglycerinether mit einem Molgewicht bis zu 1000 Dalton, 1,2-Propandiol, 1,3-Propandiol, Glycerin, 1,2-Butandiol, 1,3-Butandiol, 1,4-Butandiol, 1,2,3-Butantriol, 1,2,4-Butantriol, Pentandiole, beispielsweise 1,2-Pentandiol, 1,5-Pentandiol, Hexandiole, 1,2-Hexandiol, 1,6-Hexandiol, 1,2,6-Hexantriol, 1,4-cyclo-Hexandiol, 1,2-cyclo-Hexandiol, Heptandiole, 1,2-Heptandiol, 1,7-Heptandiol, Oktandiole, 1,2-Oktandiol, 1,8-Oktandiol, 2-Ethyl-1,3-hexandiol, Octadienole, Decadienole, Dodekandiole, 1,2-Dodekandiol, 1,12-Dodekandiol, 1,12-Dodekandiol mit 10 Mol EO, Dodecadienole.

Die erfindungsgemäßen Polyhydroxyverbindungen sind in den Zusammensetzungen in Konzentrationen von 0,01 Gew.% bis zu 20 Gew.%, vorzugsweise von 0,05 Gew.% bis zu 15 Gew.% und ganz besonders bevorzugt in Mengen von 0,1 Gew.% bis zu 10 Gew.% enthalten.

In einer weiteren Ausführungsform sollten die erfindungsgemäßen Mittel zusätzlich mindestens einen UV-Lichtschutzfilter enthalten. Unter UV-Lichtschutzfiltern sind organische Substanzen zu verstehen, die in der Lage sind, ultra-violette Strahlen zu absorbieren und die aufgenommene Energie in Form längerwelliger Strahlung, z.B. Wärme wieder abzugeben. UVB-Filter können öllöslich oder wasserlöslich sein.

Ferner können die kosmetischen Mittel weitere Wirk-, Hilfs- und Zusatzstoffe enthalten, wie beispielsweise
- Strukturanten wie Maleinsäure und Milchsäure,
- Parfümöle, Dimethylisosorbid und Cyclodextrine,
- Farbstoffe zum Anfärben des Mittels,
- Antischuppenwirkstoffe wie Piroctone Olamine, Zink Omadine und Climbazol,
- Cholesterin,
- Komplexbildner wie EDTA, NTA, β-Alanindiessigsäure und Phosphonsäuren,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher verwiesen.

Wie bereits erwähnt, kommt der hohen Pflegewirkung der erfindungsgemäßen Mittel insbesondere daher Bedeutung zu, als sie auch in Gegenwart von Oxidationsmitteln - beispielsweise im Rahmen der oxidativen Haarfärbung - hervorragende Ergebnisse liefert.

Ein zweiter Erfindungsgegenstand ist daher ein Verfahren zur Haarbehandlung, in dem ein kosmetisches Mittel gemäß Anspruch 1 auf das Haar aufgetragen wird und dort bis zur nächsten Haarwäsche verbleibt.

Erfindungsgemäß kann, obwohl dies nicht bevorzugt ist, die erfindungsgemäße Zusammensetzung auch nach einer Einwirkzeit wieder aus dem Haar ausgespült werden. Die Einwirkungszeit beträgt bevorzugt wenige Sekunden bis 100 Minuten, besonders bevorzugt 1 bis 50 Minuten und ganz besonders bevorzugt 1 bis 30 Minuten.

Die folgenden Beispiele sollen den Gegenstand der vorliegenden Erfindung erläutern ohne ihn jedoch zu beschränken.

Ein dritter Erfindungsgegenstand ist, daß die kosmetische Zusammensetzung als Non-Aerosol Sprühkur vorliegt und auf dem Haar aufgetragen wird und dort bis zur nächsten Haarwäsche verbleibt.

### Beispiele

Alle Mengenangaben sind, soweit nicht anders vermerkt, Gewichtsteile.

### Conditioner

| | C1 | C2 | C3 | C4 | C5 | V1 | V2 |
|---|---|---|---|---|---|---|---|
| Cyclomethicone and Dimethiconol | 70,0 | 70,0 | 35,0 | 70,0 | 70,0 | 70,0 | 70,0 |
| Octamethyltrisiloxane | 27,5 | ---- | ---- | 27,5 | ---- | 27,5 | ---- |
| Cyclopentasiloxane | ---- | 27,5 | 37,0 | 0 | 27,5 | 0 | 27,5 |
| Natürliches Öl | 0,5 | 0,5 | 0,5 | 0,5 | 0,5 | 0,5 | 0,5 |
| Parfüm | 0,5 | 0,5 | 0,5 | 0,5 | 0,5 | 0,5 | 0,5 |
| Farbstoff | 1,0 | 1,0 | 1,0 | 1,0 | 1,0 | 1,0 | 1,0 |
| Acrylsäureester 1 | 0,5 | 0,5 | 0,5 | ---- | ---- | ---- | ---- |
| Acrylsäureester 2 | ---- | ---- | ---- | 0,5 | ---- | ---- | ---- |
| Acrylsäureester 3 | ---- | ---- | ---- | ---- | 0,5 | ---- | ---- |
| Ethanol | ---- | ---- | ad 100 | ---- | ---- | ad 100 | ad 100 |

Die verwendeten Acrylsäureester entsprechen alle der Formel (b1), in welcher die Reste R1 bis R4 die folgende Bedeutung haben:

| | R1 | R2 | R3 | R4 |
|---|---|---|---|---|
| Acrylsäureester 1 | Phenyl | Phenyl | Cyano | 2-Ethylhexyl |
| Acrylsäureester 2 | Wasserstoff | Methoxyphenyl | Wasserstoff | 2-Ethylhexyl |
| Acrylsäureester 3 | Wasserstoff | Methoxyphenyl | Wasserstoff | Isoamyl |

Die zuvor genannte Rezepturen wurden mit 5 unterschiedlichen Parfümölen bei 45 °C in einem Lichtschrank gelagert.Die Rezepturen C1 bis C5 waren dabei jeweils mindestens 4 Wochen ohne Eintrübung, Verfärbung oder Veränderung des Duftes stabil. Die Rezepturen C1 bis C3 waren sogar 6 Wochen bei 45 °C ohne jegliche Veränderung von Farbe oder Duft stabil. Die Zusammensetzungen V1 und V2 zeigten bereits nach einer Woche deutliche starke Verfärbungen, waren nicht mehr klar und der Duft veränderte sich merklich.

## Patentansprüche

1. Kosmetische Zusammensetzung zur Behandlung von keratinischen Fasern, enthaltend
a. mindestens ein Esteröl in einer Gesamtmenge von 0,01 bis 20,0 Gew.%,
b. mindestens ein Silikon in einer Gesamtmenge von 0,01 bis 99 Gew.%,
c. mindestens ein Acrylsäurederivat der Formel c) in einer Gesamtmenge von 0,01 bis 5,0 Gew.% 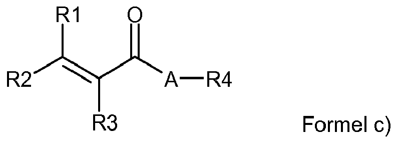 in welcher die Reste R1, R2, R3 jeweils unabhängig voneinander stehen für einen Rest ausgewählt aus Wasserstoff, einen linearen und/oder verzweigten, gesättigten und/oder ungesättigten C1 bis C30 Alkyl- oder Alkenylresten, insbesondere Methyl, Ethyl, Propyl, i-Propyl, Butyl, i-Butyl, t-Butyl, Pentyl, Amyl, i-Amyl, neo-Pentyl, Hexyl, i-Hexyl, 2-Ethylhexyl, Heptanyl, i-Heptanyl, Octanyl, i-Octanyl, Nonyl, Decyl, Lauryl, Myristyl, Aryl, Phenyl, Methoxyphenyl, Hydroxyphenyl, Methoxybenzyl, Hydroxybenzyl, Benzyl, Naphthyl oder-CN,
A steht für O oder-NR5 und
R4 sowie gegebenenfalls R5 stehen unabhängig voneinander für einen Rest ausgewählt aus Wasserstoff, einen linearen und/oder verzweigten, gesättigten und/oder ungesättigten C1 bis C30 Alkyl- oder Alkenylresten, insbesondere Methyl, Ethyl, Propyl, i-Propyl, Butyl, i-Butyl, t-Butyl, Pentyl, Amyl, i-Amyl, neo-Pentyl, Hexyl, i-Hexyl, 2-Ethylhexyl, Heptanyl, i-Heptanyl, Octanyl, i-Octanyl, Nonyl, Decyl, Lauryl, Myristyl, Aryl, Phenyl, Methoxyphenyl, Hydroxyphenyl, Methoxybenzyl, Hydroxybenzyl, Benzyl oder Naphthyl, und
d. Wasser in einer Gesamtmenge von 0 bis höchstens 5,0 Gew.%.

2. Kosmetische Zusammensetzung nach Anspruch 1, **dadurch gekennzeichnet, daß** es, mindestens ein Silikon ausgewählt aus der Gruppe der Dimethicone und/oder und der Gruppe der Dimethiconole und/oder der Cyclomethicone enthält.

3. Kosmetische Zusammensetzung nach einem der Ansprüche 1 oder 2, **dadurch gekennzeichnet, dass** in der Formel c) A steht für ein Sauerstoffatom.

4. Kosmetische Zusammensetzung nach einem der Ansprüche 1 bis 3, **dadurch gekennzeichnet, daß** das Esteröl ausgewählt ist aus den pflanzlichen und/oder natürlichen Ölen.

5. Kosmetische Zusammensetzung nach einem der Ansprüche 1 bis 4, **dadurch gekennzeichnet, daß** das Esteröl ausgewählt ist aus Amaranthsamenöl, Aprikosenkernöl, Arganöl, Avocadoöl, Babassuöl, Baumwollsaatöl, Granatapfelkernöl, Grapefruitsamenöl, Haselnussöl, Holundersamenöl, Johannesbeersamenöl, Jojobaöl, Kakaobutter, Macadamianussöl, Maiskeimöl, Mandelöl, Marulaöl, Nachtkerzenöl, Olivenöl, Orangenöl, Sanddornfruchtfleischöl, Sanddornkernöl, Sesamöl, Sheabutter, Sojaöl, Sonnenblumenöl, Traubenkernöl, Walnußöl Weizenkeimöl, Wildrosenöl und deren Mischungen.

6. Kosmetische Zusammensetzung nach einem der Ansprüche 1 bis 5, **dadurch gekennzeichnet, daß** es zusätzlich mindestens einen Stoff aus der Gruppe der Vitamine, Provitamine und Vitaminvorstufen sowie deren Derivate enthält, wobei Vitamine, Pro-Vitamine und Vitaminvorstufen bevorzugt sind, die den Gruppen A, B, C, E, F und H zugeordnet werden.

7. Kosmetische Zusammensetzung nach einem der Ansprüche 1 bis 6, **dadurch gekennzeichnet, dass** es zusätzlich mindestens einen Stoff aus der Gruppe der kationischen Tenside und/oder der kationischen Polymere enthält.

8. Kosmetische Zusammensetzung nach einem der Ansprüche 1 bis 7, **dadurch gekennzeichnet, dass** der Wassergehalt höchstens 0,3 Gew.% beträgt

9. Verwendung einer Zusammensetzung nach einem der Ansprüche 1 bis 8 zur Verbesserung der Lagerstabilität der Zusammensetzung.

10. Verfahren zur Behandlung von Haar, bei dem eine Zusammensetzung gemäß einem der Ansprüche 1 bis 8 auf das Haar aufgetragen wird und dort bis zur nächsten Haarwäsche verbleibt.
